# EUROPEAN PATENT APPLICATION

(11) **EP 2 992 885 A2**
(43) Date of publication of application: **09.03.2016**
(21) Application number: 15182424.0
(22) Date of filing: 25.08.2015
(51) Int. Cl.: A61K 31/496, A61K 31/522, A61P 3/06

(54) **METHOD FOR INHIBITING A LIVER DISEASE**

(30) Priority: 02.09.2014 US 201462044593 P
(71) Applicant: Jansfat Biotechnology Co., Ltd., Kaohsiung City 807 (TW)
(72) Inventor: Chen, Ing-Jun, Kaohsiung City (TW)
(74) Representative: Lippert, Stachow & Partner

(57) **Abstract**

A method for inhibiting a liver disease is provided. The method includes administering a pharmaceutical composition of one of a piperazine analogue and a piperazine analogue complex to a warm-blooded animal suffering from the liver disease.

## Description

The application claims the benefit of US provisional patent application Ser. No.62/044,593, filed on September 2, 2014, the entire contents of which all are hereby incorporated by reference.

The present invention is related to a combination therapeutic method using a pharmaceutical composition of a Piperazinyl derivative and a different active agent capable of preventing non-alcoholic fatty liver disease, hyperadiposity and reducing high-fat diet-induced accumulation of fat in the liver.

Caffeine has been used to treat obesity by increasing energy expenditure and thermogenesis in brown adipose tissue through uncoupling protein 1 and adrenergic activation. However, muscle-uncoupling protein 3 expression is unchanged even after chronic ephedrine/caffeine treatment. KMUP-1 has increased endothelium nitric oxide synthase (eNOS)/cGMP as sildenafil, but the latter has a lack of G-protein-coupled receptors (GPCRs) antagonist activity. Coffee intake was shown to be associated with lower rates of liver disease and reduced risk of hepatocellular carcinoma and liver cirrhosis.

Previously, xanthine-based KMUP-1 has been proven to relax vascular and airway smooth muscle contractions by enhancing eNOS /guanosine 3',5'-cyclic monophosphate (cGMP)-pathway, including increasing soluble guanylyl cyclase α1 (sGCα1) and protein kinase G (PKG) expression.

KMUP-1 and sildenafil increase cGMP/PKG in lipid metabolism and have been used as phosphodiesterase type 5A (PDE-5A) inhibitors and eNOS-enhancers, but their effects on obesity were little studied. An increase of cGMP can promote the expansion and lowering of white adipose tissues, unlike visceral epididymal fat pads. Indeed, eNOS and HSL in obesity are related to decreased subcutaneous adipose tissue lipolysis; the inhibition of NOS resulted in increased lipolysis in white tissues and the addition of nitric oxide (NO) caused the inhibition of lipolysis.

In accordance with an aspect of the present invention, a method for inhibiting a liver disorder caused by lipid accumulation in a subject is provided. The method includes a step of administering to the subject suffering the disorder an effective amount of a composition characterized by comprising an ingredient being one selected from the group consisting of a KMUPs-RX complex compound and a KMUPs-RX-RX complex compound to the subject in a suitable dosage for animals.

In accordance with another aspect of the present invention, a method for treating a disorder caused by liver disease is disclosed. The method includes steps of providing a subject in need thereof; and administering one selected from the group consisting of a Sildenafil Analogs derivative compound and a Sildenafil Analogs-RX complex compound, pharmaceutically acceptable salts thereof; and a pharmaceutical composition thereof to the subject in an oral dosage suitable for animals.

In accordance with a further aspect of the present invention, a method for treating a liver disease is disclosed. The method includes a step of administering a therapeutically effective amount of a compound being one selected from the group consisting of Piperazinyl Analogs and Piperazinyl Complex Analogs to a warm-blooded animal having the disease including one selected from the group consisting of a liver disease, non-alcoholic fatty liver disease, hyperadiposity, hepatic steaotosis, HFD-induced obesity, lipid accumulation combined with inflammation, setting the stage for further liver damage, and a combination thereof.

In accordance with further another aspect of the present invention, a method for treating a liver disease is disclosed. The method includes a step of administering a therapeutically effective amount of a pharmaceutical composition, in which the active agent is one selected from the group consisting of a Sildenafil Analogs derivative compound and a Sildenafil Analogs-RX complex compound.

A further aspect for the combination is to administer an effective amount of a Piperazinyl Analogs compound and an other different active agent to a warm-blooded animal in need thereof.

A further aspect for the combination is to administer a therapeutically effective amount of a pharmaceutical composition, in which the active agent is a Sildenafil Analogs compound and different active agent to a warm-blooded animal in need thereof.

The liver disease-inhibiting pharmaceutical composition includes:
an effective amount of a KMUPs complex compound represented by formula II or formula III, characterized in that
R₂ and R₄ are each selected independently from the group consisting of a C1-C5 alkoxy group, hydrogen atom, nitro group, and a halogen atom;
RX includes a carboxylic group which is donated from one of a Statin analogues, co-polymer, poly-γ-polyglutamic acid derivative, an D-ascorbic acid, L-ascorbic acid, DL-ascorbic acid, oleic acid, phosphoric acid, citric acid, nicotinic acid and sodium carboxymethyl cellulose (sodium CMC); and
RX is an anion of a carboxylic group donated from one of a statins analogues, co-polymer, poly-γ-polyglutamic acid derivative, D-ascorbic acid, L-ascorbic acid, DL-ascorbic acid, oleic acid, phosphoric acid, citric acid, nicotinic acid and sodium carboxymethyl cellulose (sodium CMC); and
a pharmaceutically acceptable carrier.

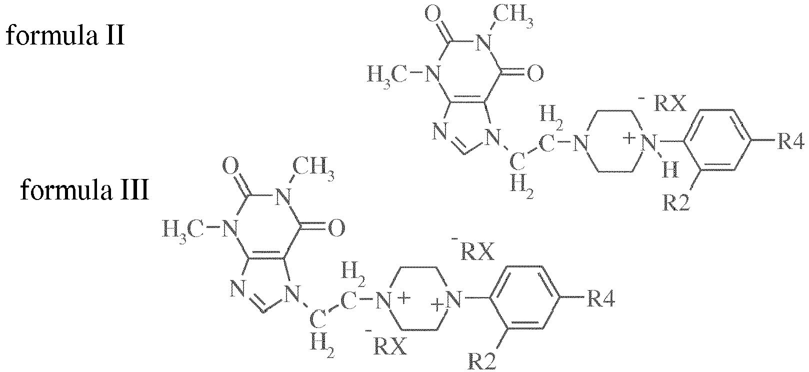

The objectivs and advantages of the present invention will become more readily apparent to those ordinarily skilled in the art after reviewing the following detailed descriptions and accompanying drawings, in which:

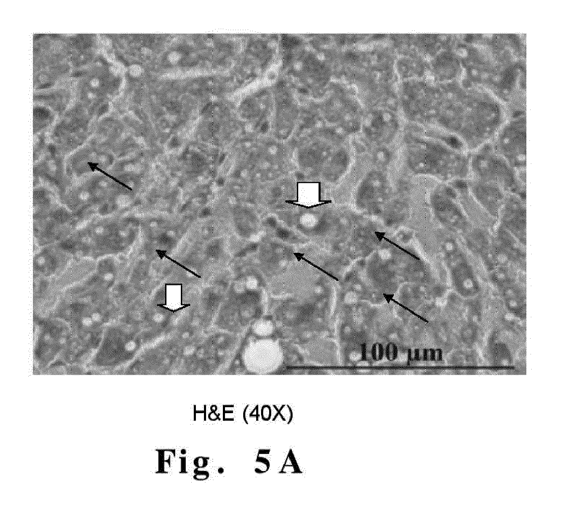

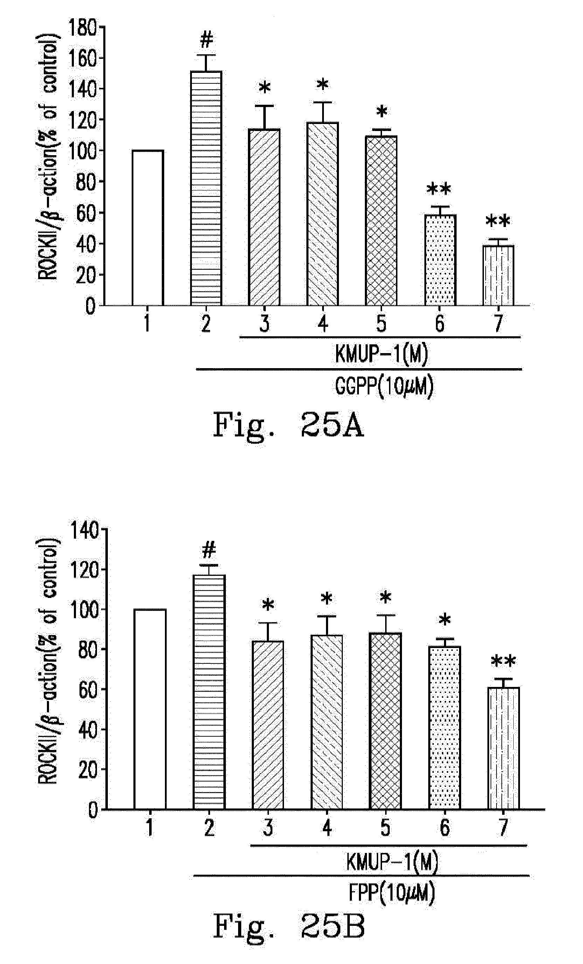

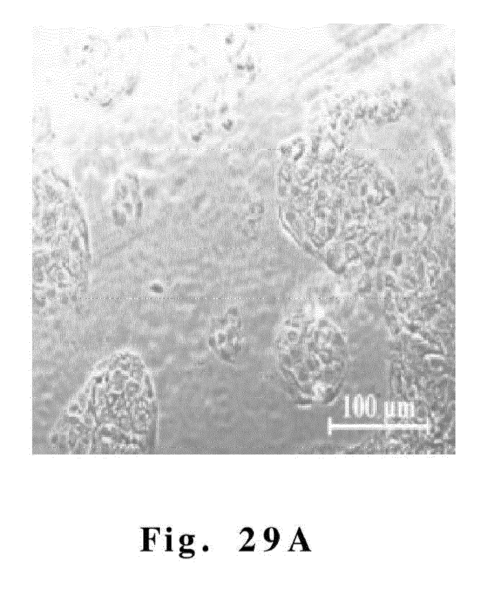

The patent application contains at least one drawing executed in color. Copies of this patent or patent application publication with color drawings will be disclosed by the Office upon request with payment of the necessary fee.
Fig. 1 shows the effects of KMUP-1 accompanied by HFD-induced body/weight changes.
Fig. 2A-2C show the morphology of the liver.

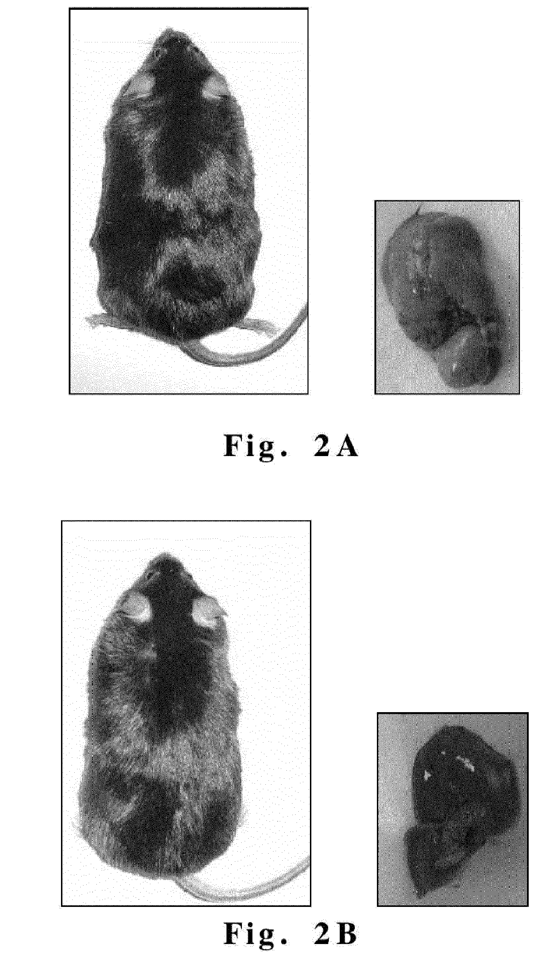

Fig. 2A shows the morphology of a liver fed with HFD.
Fig. 2B shows the morphology of mice treated with KMUP-1.

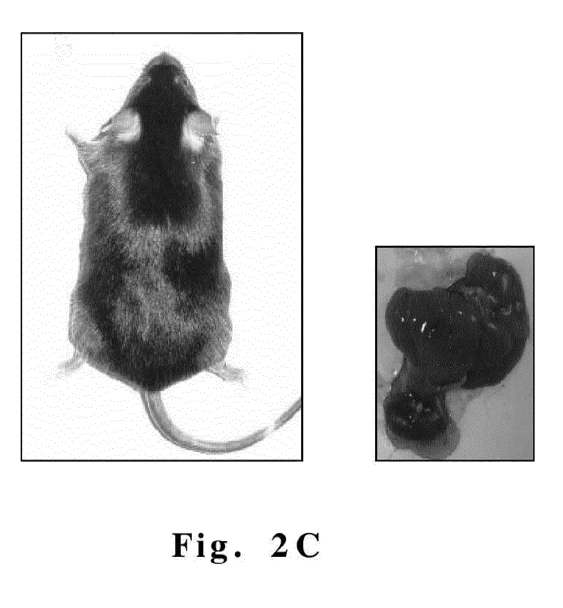

Fig. 2C shows the morphology of the mice protection group.

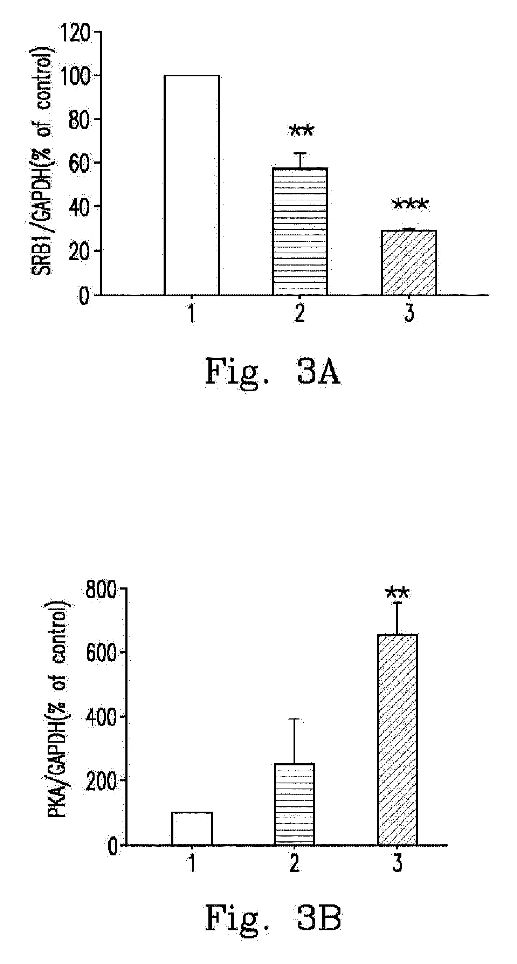

Fig. 3A-3B show the effects of KMUP-1 on HFD-induced hepatic SR-B1 and PKA expression.
Fig. 3A shows the effects of KMUP-1 on SR-B1 expression.
   1... HFD group 2 ... treatment group
   3.... protection group (KMUP-1 HCl, 2.5 mg/200 ml)
Fig. 3B shows the effects of KMUP-1 on PKA expression.
   1... HFD group 2 ... treatment group
   3.... protection group (KMUP-1 HCl, 2.5 mg/200 ml)

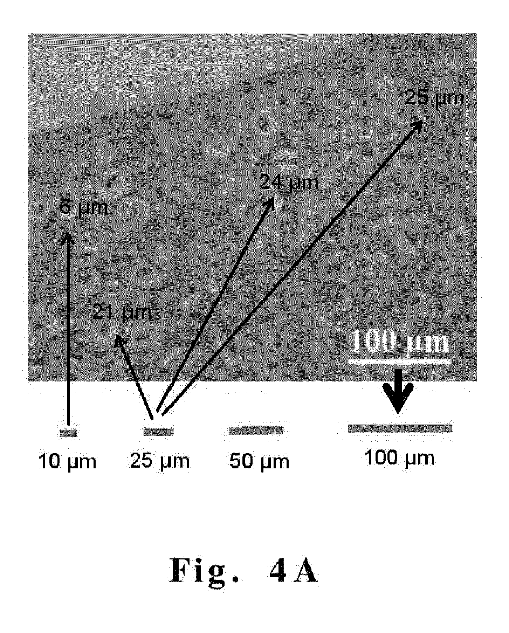

Fig. 4A-4B show the morphologic demonstration of a cross-section of oil globule accumulation in a liver.
Fig. 4A shows the demonstration standards for fat globules.

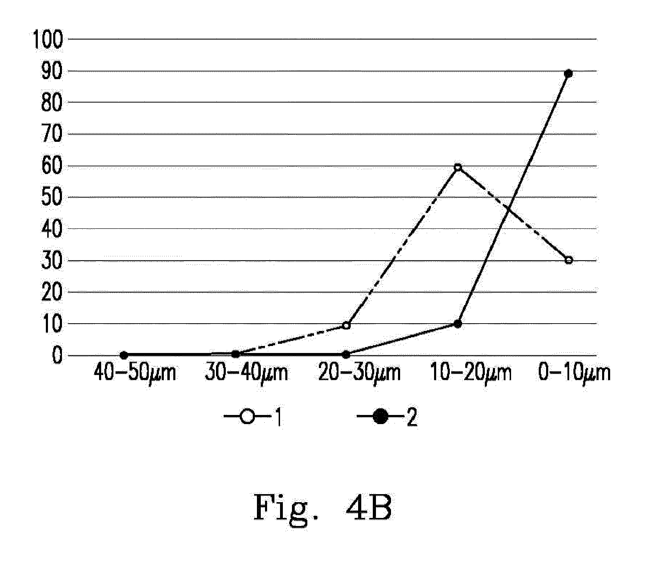

Fig. 4B shows the distribution of diameter changes and number of oil globulets.
   1... HFD group 2 ... KMUP-1 protection group
Fig. 5A-5C show the gross morphology of an inflamed liver.
Fig. 5A shows the morphologic demonstration of fat globules and Mallory's hyaline bodies.

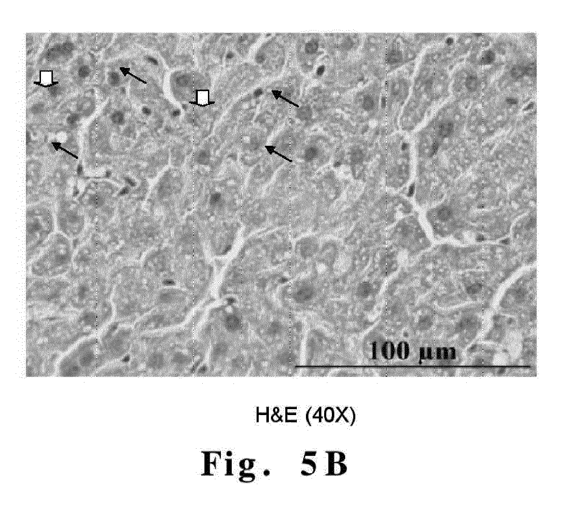

Fig. 5B shows the gross morphology of the liver protection group.

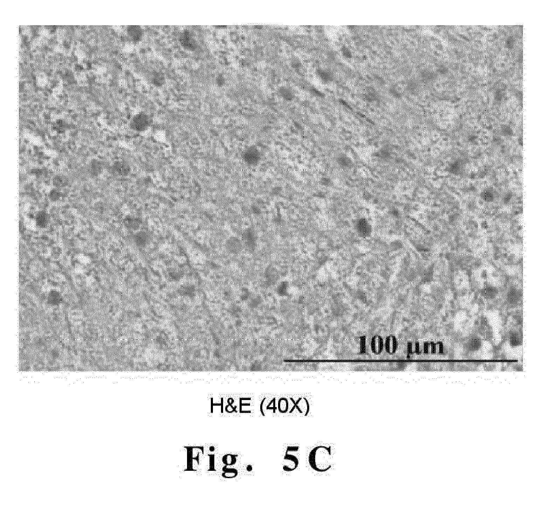

Fig. 5C shows the gross morphology of the liver treatment group.

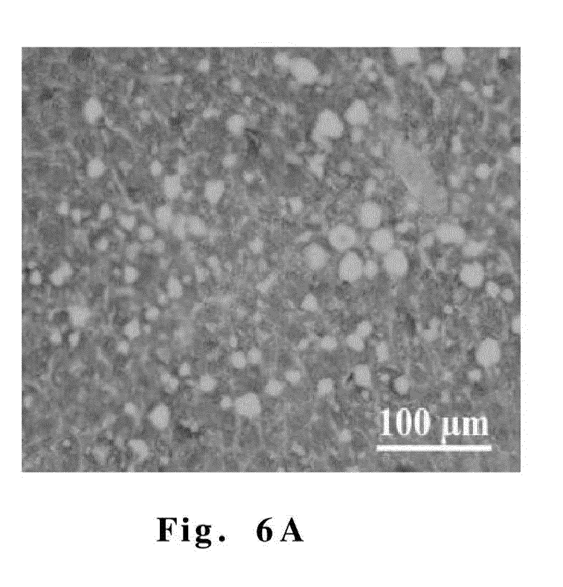

Fig. 6A-6C show the IHC staining of inflammatory TNF-α.
Fig. 6A shows the IHC staining on fatty liver slices from HFD mice.

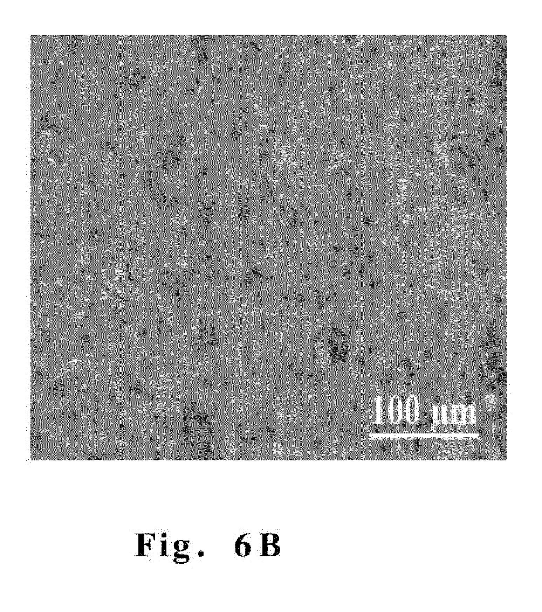

Fig. 6B shows the treatment group.

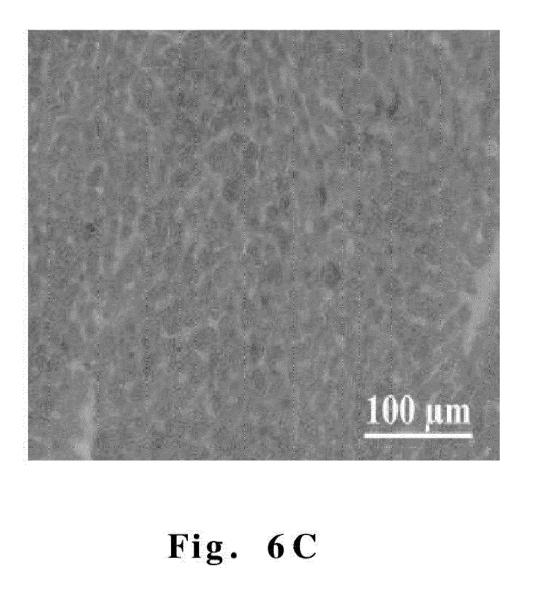

Fig. 6C shows the protection group.

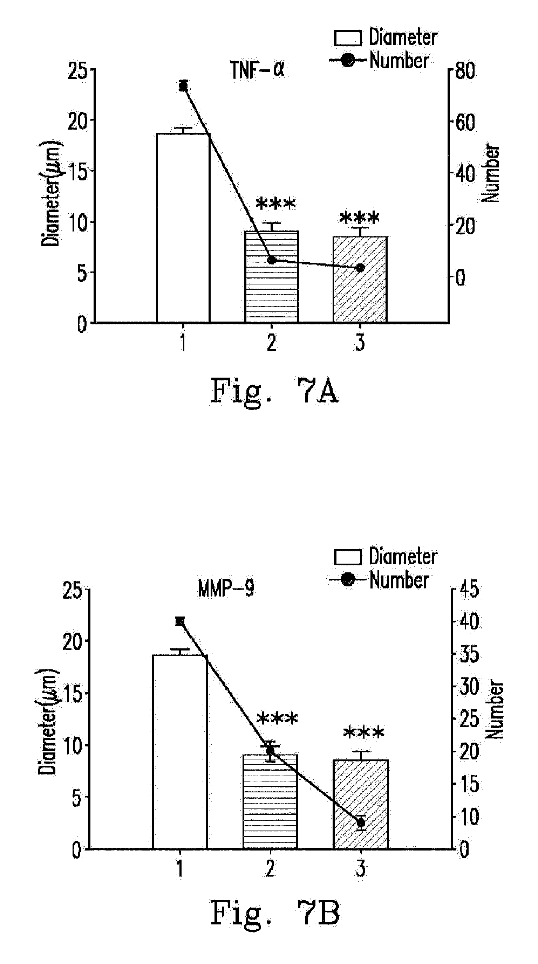

Figs. 7A-7E show the oil globulets diameter and changes in globulet number in a liver.
   1... HFD group 2 ... treatment group
   3.... protection group
Fig. 7A shows the IHC staining of TNF-α □ in HFD-induced liver steatosis.
Fig. 7B shows the IHC staining of MMP-9 in HFD-induced liver steatosis.

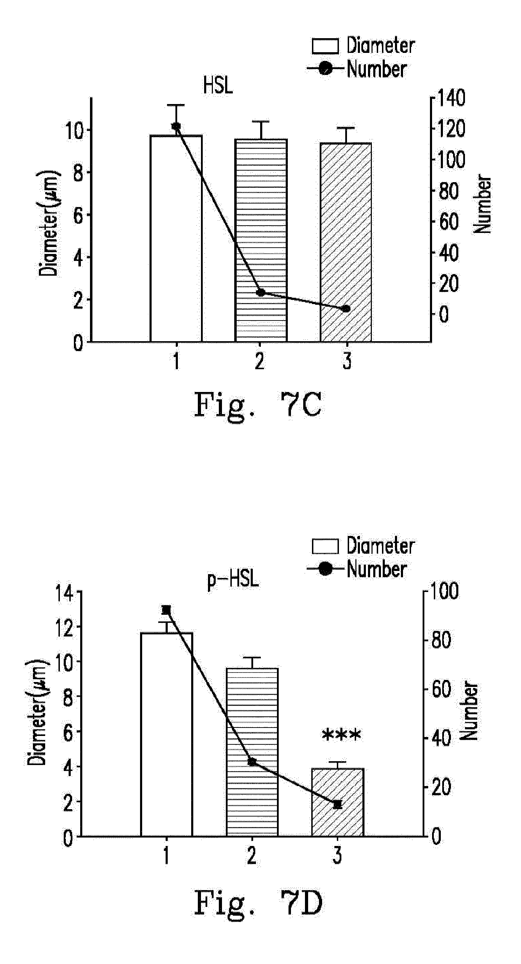

Fig. 7C shows the IHC staining of HSL □ in HFD-induced liver steatosis.

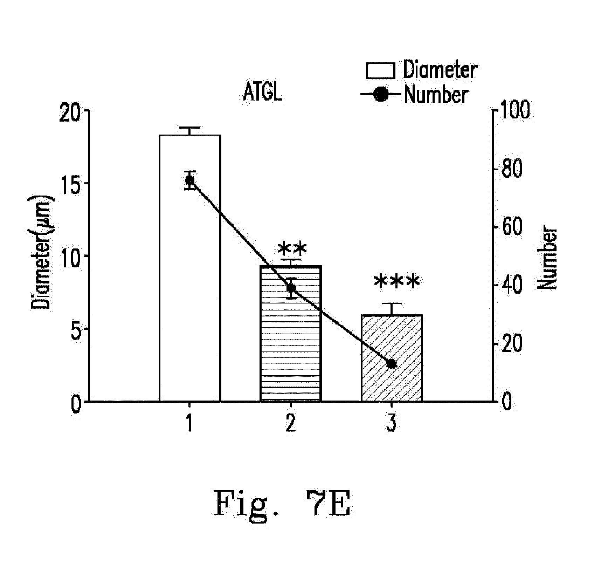

Fig. 7E shows the IHC staining of ATGL in HFD-induced liver steatosis.

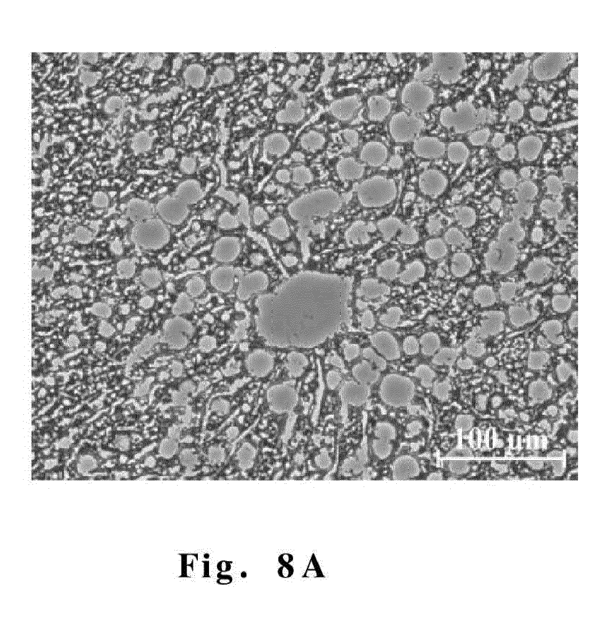

Figs. 8A-8C show the CD11c staining for the M1 type.
Fig. 8A shows the CD11c staining for the M1 type of HFD mice.

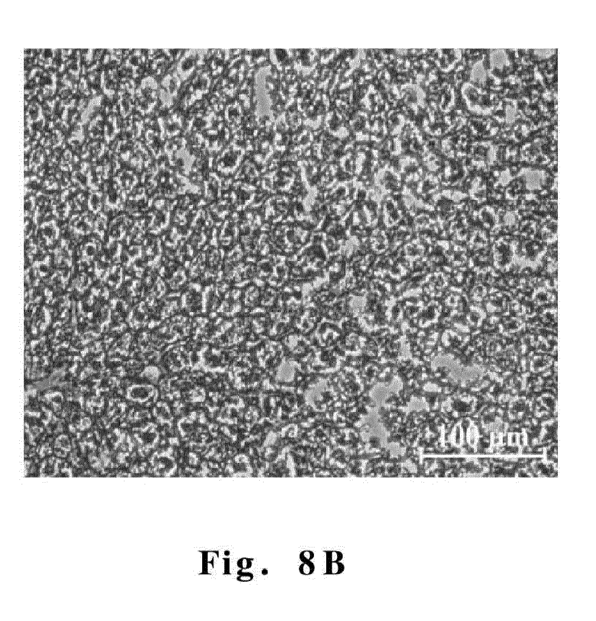

Fig. 8B shows the treatment group.

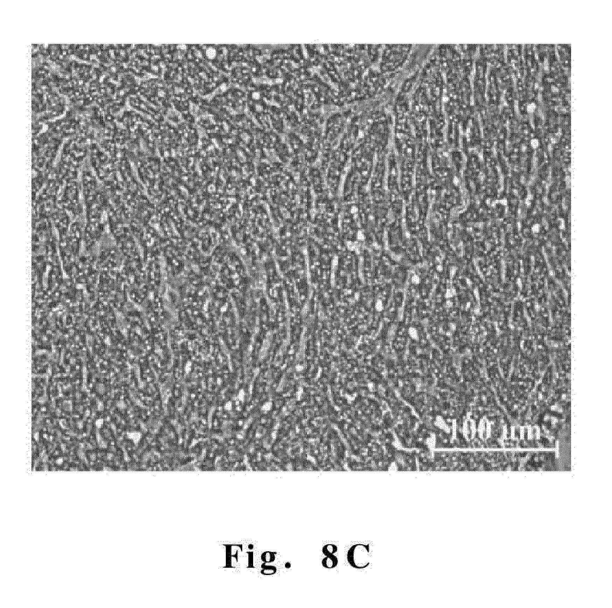

Fig. 8C shows the protection group.

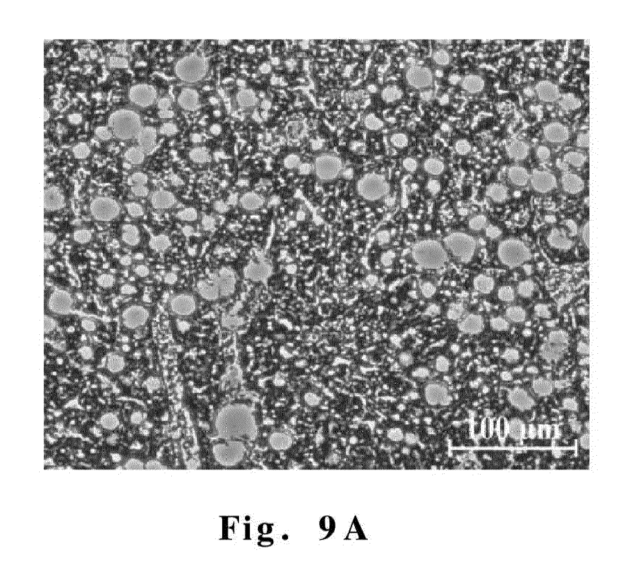

Figs. 9A-9C show the CD209a staining for the M2 type.
Fig. 9A shows the CD209a staining for the M2 type of HFD mice.

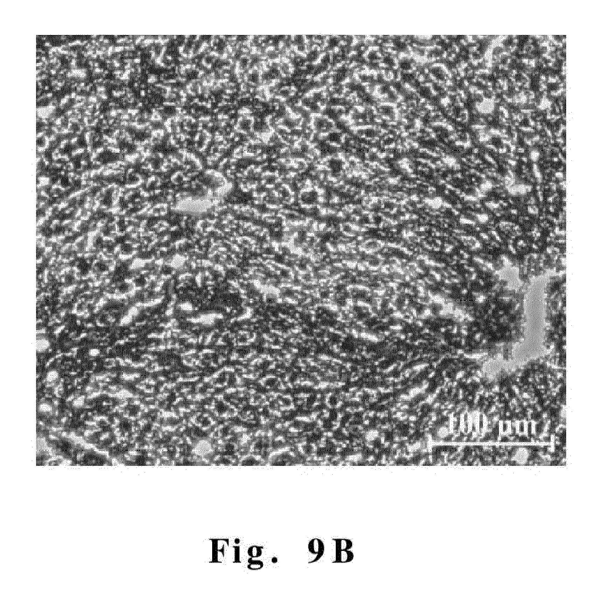

Fig. 9B shows the protection group.

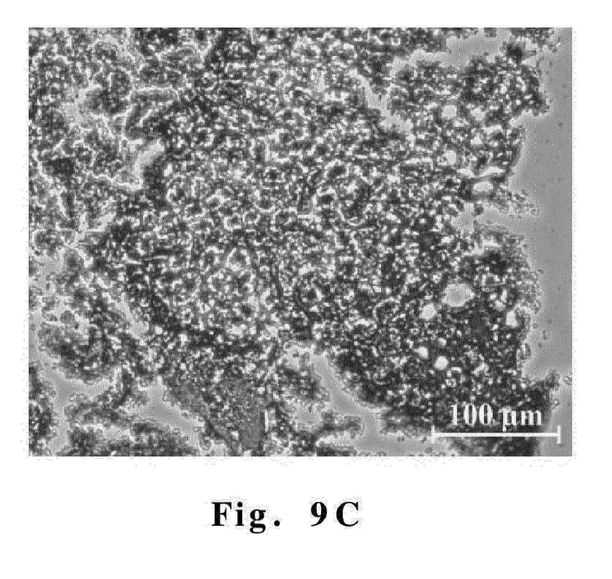

Fig. 9C shows the treatment group.

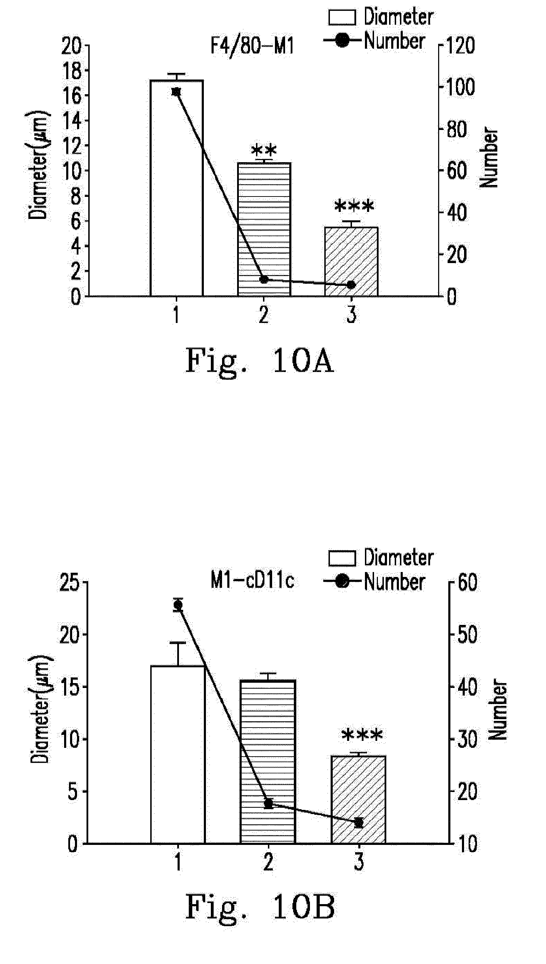

Figs. 10A-10B show the oil globulets diameter and changes in globulet number for the M1 type.
   1... HFD group 2 ... treatment group
   3.... protection group
Fig. 10A shows the F4/80 staining for the M1 type.
Fig. 10B shows the CD11c staining for the M1 type.

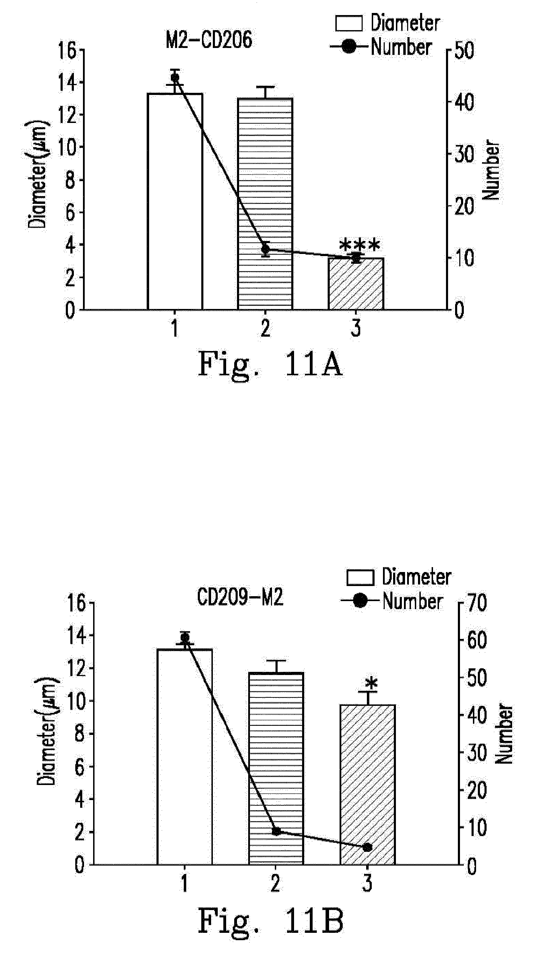

Figs. 11A-11B show the oil globulets diameter and changes in globulet number for the M2 type.
   1... HFD group 2 ... treatment group
   3.... protection group
Fig. 11A shows the CD206 staining for the M2 type.
Fig. 11B shows the CD209a staining for the M2 type.

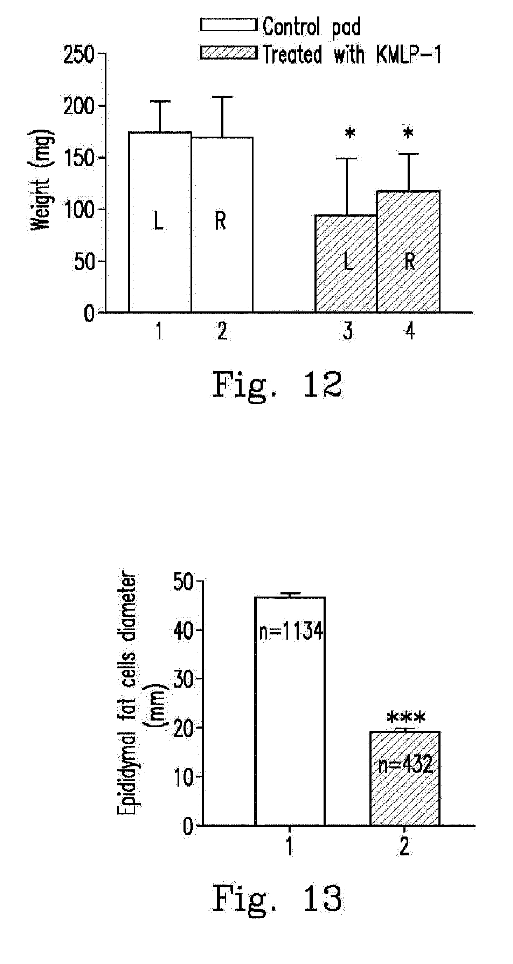

Figs. 12 show the effects of KMUP-1 reduced epididymal fat pad weight of HFD mice.
   1...control group left epididymal fat pad side
   2...control group right epididymal fat pad side
   3.... treatment group left epididymal fat pad side (KMUP-1)
   4.... treatment group right epididymal fat pad side (KMUP-1)
Fig. 13 shows the effects of KMUP-1 reduced fat cell diameter of HFD mice.
   1...control group 2...treatment group (KMUP-1)

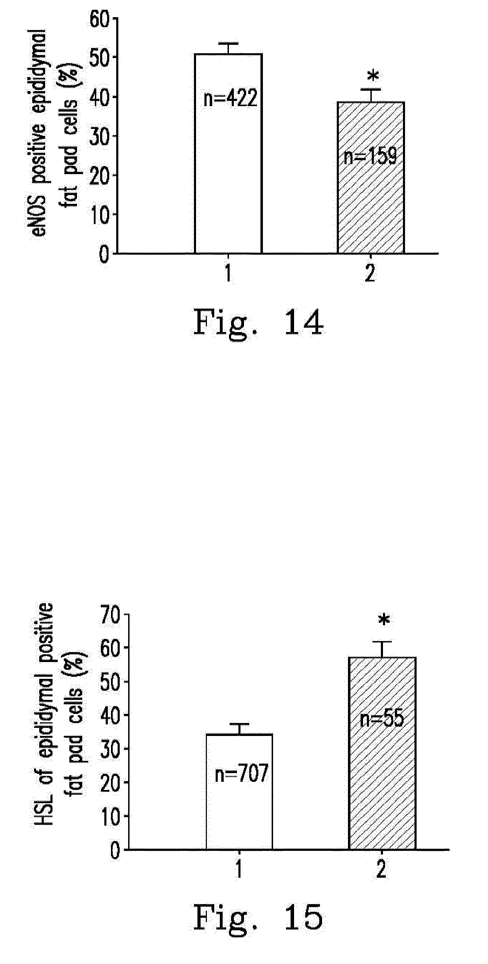

Fig. 14 shows the effects of KMUP-1 resulting the changes of eNOS in epididymal fat pads.
   1...control group 2...treatment group (KMUP-1)
Fig. 15 shows the effects of KMUP-1 resulting the changes of HSL in epididymal fat pads.
   1...control group 2...treatment group (KMUP-1)

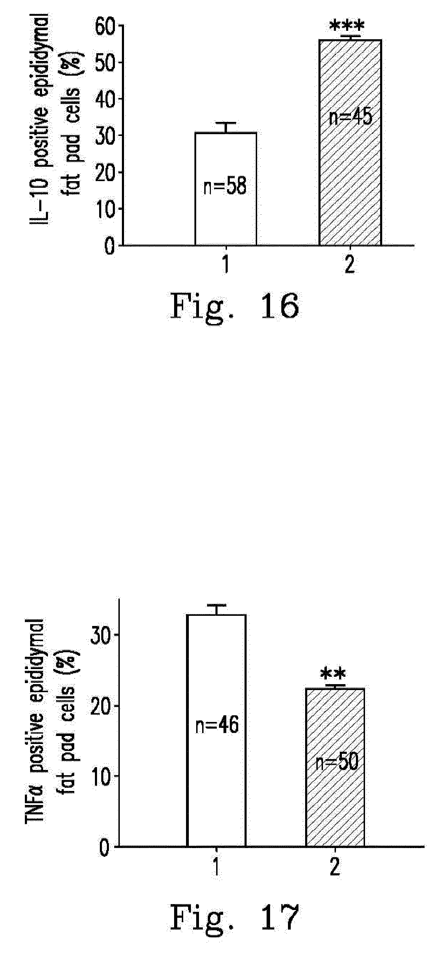

Fig. 16 shows the effects of KMUP-1 resulting the changes of IL-10 in epididymal fat pads.
   1...control group 2...treatment group (KMUP-1)
Fig. 17 shows the effects of KMUP-1 resulting the changes of TNF-α in epididymal fat pads.
   1...control group 2...treatment group (KMUP-1)

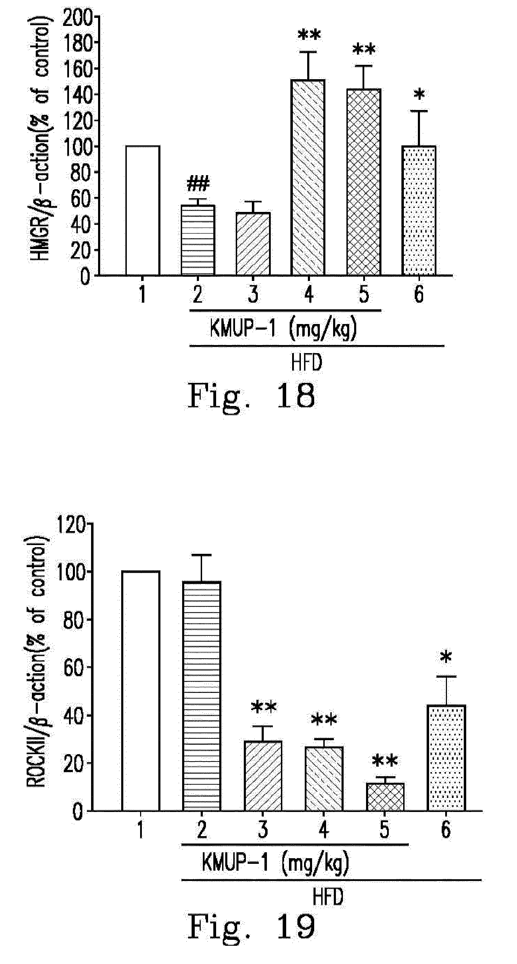

Fig. 18 shows the effects of KMUP-1 resulting in the changes of HMG CoA reductase expression in liver.
   1...control group
   2...treatment group (KMUP-1, positive control)
   3...treatment group (KMUP-1, 1 mg/kg)
   4...treatment group (KMUP-1, 2.5 mg/kg)
   5...treatment group (KMUP-1, 5 mg/kg)
   6....treatment group (simvastatin, 5 mg/kg)
Fig. 19 shows the effects of KMUP-1 resulting the changes of ROCK II expression in liver.
   1...control group
   2...treatment group (KMUP-1, positive control)
   3...treatment group (KMUP-1, 1 mg/kg)
   4...treatment group (KMUP-1, 2.5 mg/kg)
   5...treatment group (KMUP-1, 5 mg/kg)
   6....treatment group (simvastatin, 5 mg/kg)

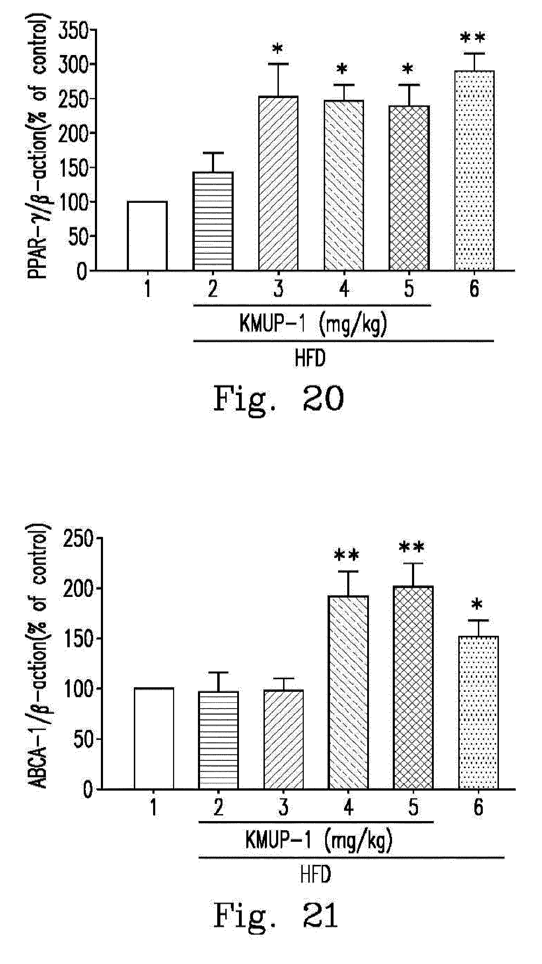

Fig. 20 shows the effects of KMUP-1 resulting in the changes of PPARγ expression in liver.
   1...control group
   2...treatment group (KMUP-1, positive control)
   3... treatment group (KMUP-1, 1 mg/kg)
   4...treatment group (KMUP-1, 2.5 mg/kg)
   5... treatment group (KMUP-1, 5 mg/kg)
   6....treatment group (simvastatin, 5 mg/kg)
Fig. 21 shows the effects of KMUP-1 resulting in the changes of ABCA1 expression in the liver.
   1...control group
   2...treatment group (KMUP-1, positive control)
   3... treatment group (KMUP-1, 1 mg/kg)
   4...treatment group (KMUP-1, 2.5 mg/kg)
   5... treatment group (KMUP-1, 5 mg/kg)
   6....treatment group (simvastatin, 5 mg/kg)

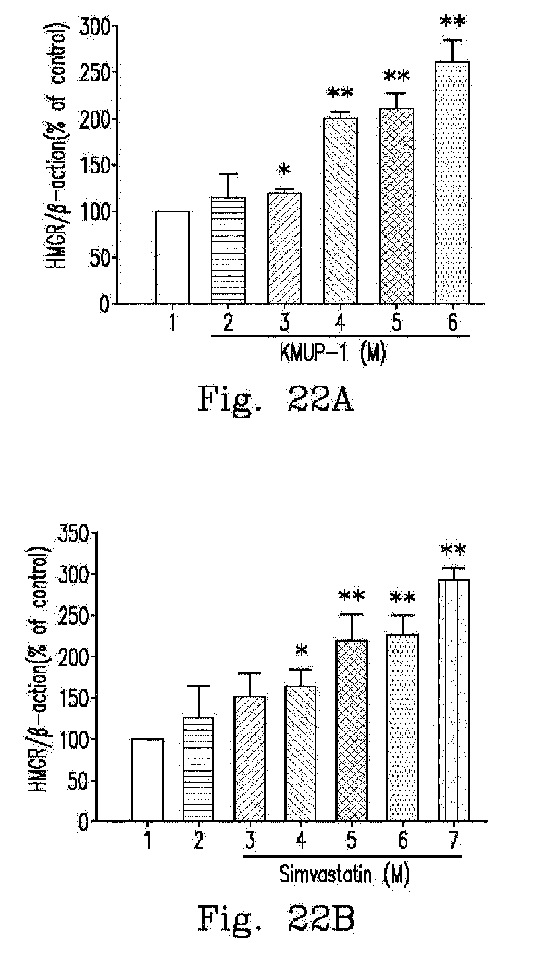

Fig. 22A-22B show the effects of KMUP-1 and simvastatin resulting in the changes of HMG CoA reductase expression in Serum.
Fig. 22A shows the effects of KMUP-1 for HMG CoA reductase expression.
   1...control group 2...treatment group (KMUP-1, 10⁻⁹ M)
   3...treatment group (KMUP-1, 10⁻⁸ M)
   4...treatment group (KMUP-1, 10⁻⁷ M)
   5... treatment group (KMUP-1, 10⁻⁶ M)
   6....treatment group (KMUP-1, 10⁻⁵ M)
Fig. 22B shows the effects of simvastatin for HMG CoA reductase expression.
   1...control group 2...vehicle
   3...treatment group (simvastatin, 10⁻⁹ M)
   4...treatment group (simvastatin, 10⁻⁸ M)
   5... treatment group (simvastatin, 10⁻⁷ M)
   6.... treatment group (simvastatin, 10⁻⁶ M)
   7.... treatment group (simvastatin, 10⁻⁵ M)

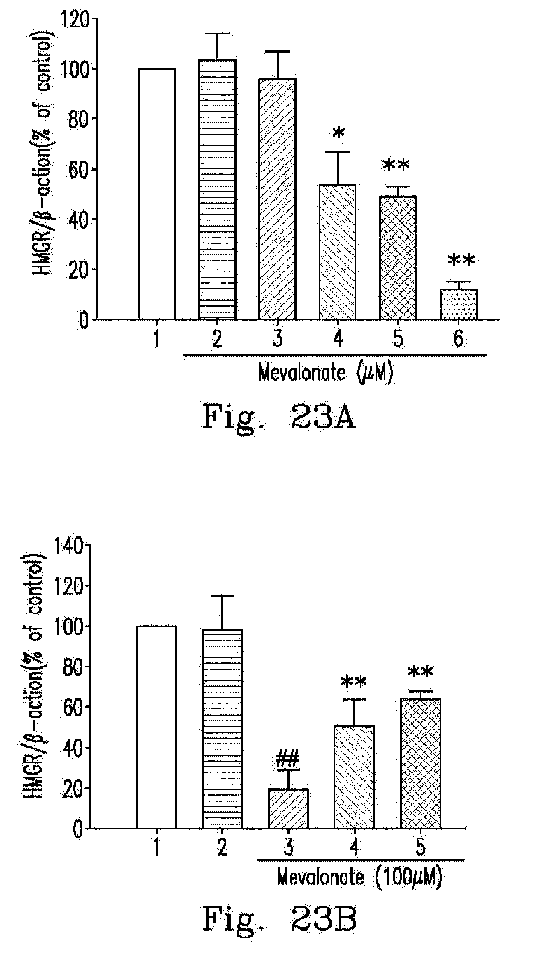

Figs. 23A-23B show the effects of mevalonate and KMUP-1 for HMG CoA reductase expression.
Fig. 23A shows the effects of mevalonate for HMG CoA reductase expression.
   1...control group 2...mevalonate, 20 µM
   3...mevalonate, 40 µM 4...mevalonate, 60 µM
   5...mevalonate, 80 µM 6....mevalonate, 100 µM
Fig. 23B shows the effects of mevalonate+ KMUP-1 for HMG CoA reductase expression.
   1...control group 2...vehicle
   3...treatment group (mevalonate, control group)
   4...treatment group (simvastatin, 10⁻⁵ M)
   5... treatment group (KMUP-1, 10⁻⁵ M)

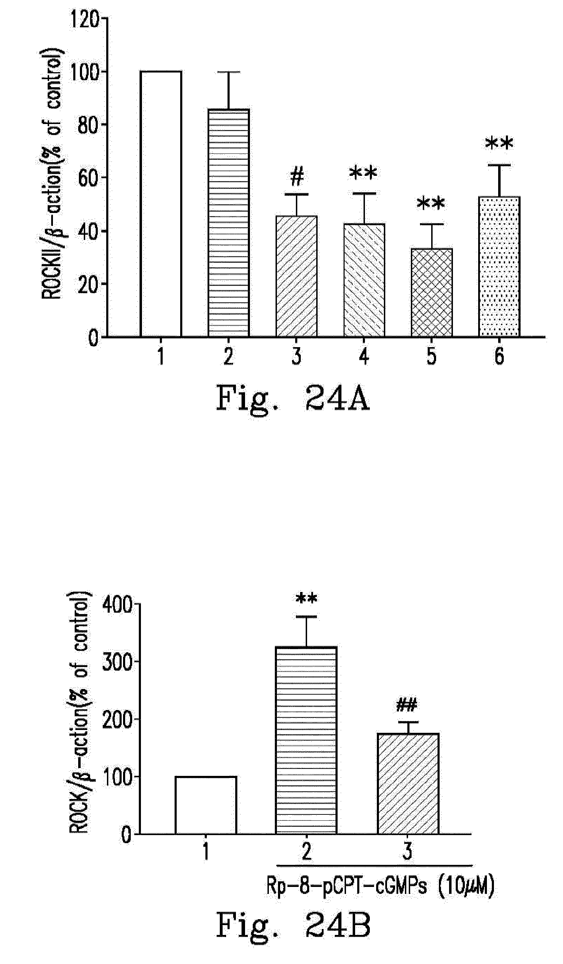

Figs. 24A-24D show the RhoA/ROCK II expression.
Fig. 24A shows the effects of C3 exoenzyme and Y27632.
   1...control group 2...vehicle
   3...simvastatin, 10⁻⁵ M 4...KMUP-1, 10⁻⁵ M
   5...C3 exoenzyme, 5 ng/ml 6....Y27632, 10⁻⁵ M
Fig. 24B shows the effects of Rp-8-pCPT-cGMPs.
   1...control group
   2...Rp-8-pCPT-cGMPs (10 µM)
   3...Rp-8-pCPT-cGMPs (10 µM) +KMUP-1(10 µM)

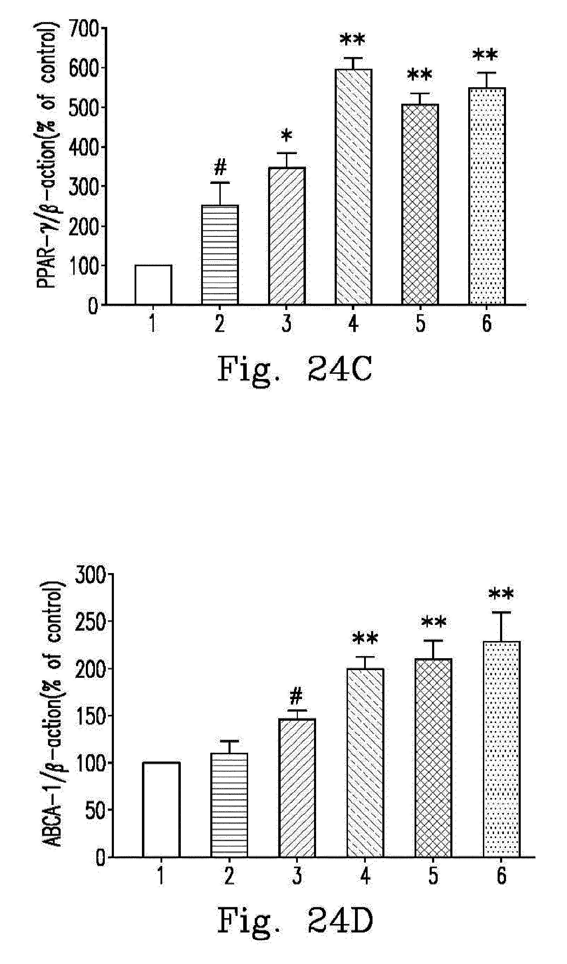

Fig. 24C shows the effects of PPARγ expression.
   1...control group 2...vehicle
   3...simvastatin, 10⁻⁵ M 4...KMUP-1, 10⁻⁵ M
   5...C3 exoenzyme, 5 ng/ml 6....Y27632, 10⁻⁵ M
Fig. 24D shows the effects of ABCA1 expression.
   1...control group 2...vehicle
   3...simvastatin, 10⁻⁵ M 4...KMUP-1, 10⁻⁵ M
   5...C3 exoenzyme, 5 ng/ml 6....Y27632, 10⁻⁵ M
Figs. 25A-25C show the RhoA/ROCK II activation.
Fig. 25A shows the effects of GGPP.
   1...control group
   2...treatment group (KMUP-1, positive control)
   3...treatment group (KMUP-1, 10⁻⁹ M)
   4...treatment group (KMUP-1, 10⁻⁸ M)
   5... treatment group (KMUP-1, 10⁻⁷ M)
   6... treatment group (KMUP-1, 10⁻⁶ M)
   7....treatment group (KMUP-1, 10⁻⁵ M)
Fig. 25B shows the effects of FPP.
   1...control group
   2...treatment group (KMUP-1, positive control)
   3...treatment group (KMUP-1, 10⁻⁹ M)
   4...treatment group (KMUP-1, 10⁻⁸ M)
   5... treatment group (KMUP-1, 10⁻⁷ M)
   6...treatment group (KMUP-1, 10⁻⁶ M)
   7....treatment group (KMUP-1, 10⁻⁵ M)

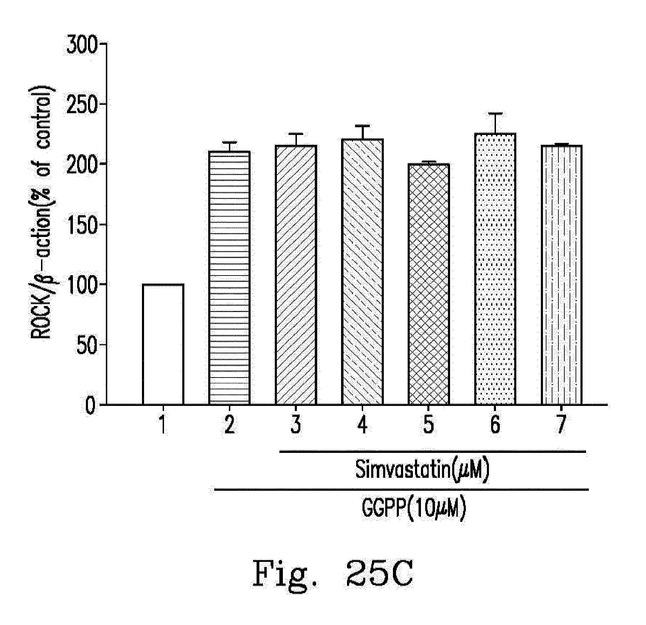

Fig. 25C shows the effects of simvastatin
   1...control group
   2...treatment group (simvastatin, positive control)
   3...treatment group (simvastatin, 10⁻⁹ M)
   4...treatment group (simvastatin, 10⁻⁸ M)
   5... treatment group (simvastatin, 10⁻⁷ M)
   6.... treatment group (simvastatin, 10⁻⁶ M)
   7.... treatment group (simvastatin, 10⁻⁵ M)

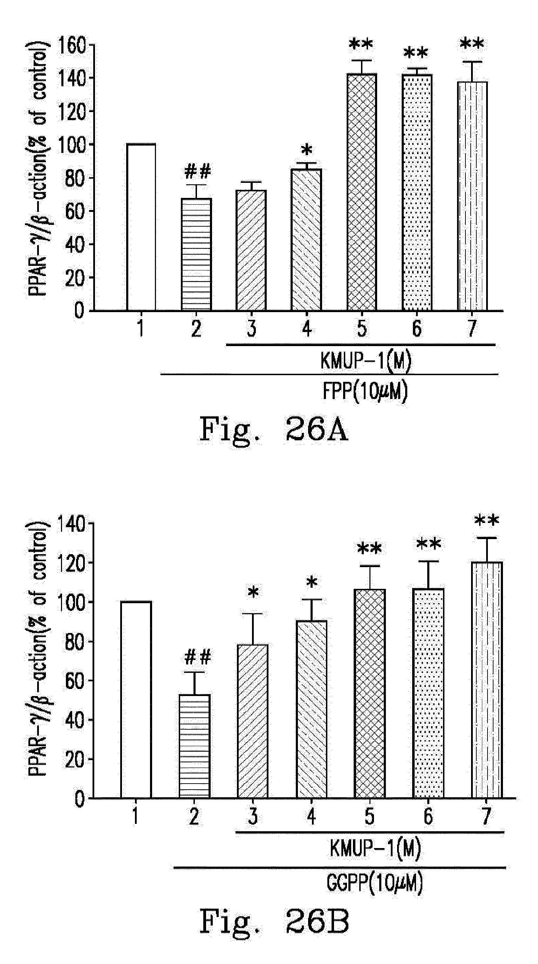

Figs. 26A-26C show the PPARγ expression.
Fig. 26A shows the effects of KMUP-1, incubation of cells with FPP alone.
   1...control group
   2...treatment group (KMUP-1, positive control)
   3...treatment group (KMUP-1, 10⁻⁹ M)
   4...treatment group (KMUP-1, 10⁻⁸ M)
   5... treatment group (KMUP-1, 10⁻⁷ M)
   6... treatment group (KMUP-1, 10⁻⁶ M)
   7....treatment group (KMUP-1, 10⁻⁵ M)
Fig. 26B shows the effects of KMUP-1, incubation of cells with GGPP alone.
   1...control group
   2...treatment group (KMUP-1, positive control)
   3...treatment group (KMUP-1, 10⁻⁹ M)
   4... treatment group (KMUP-1, 10⁻⁸ M)
   5... treatment group (KMUP-1, 10⁻⁷ M)
   6...treatment group (KMUP-1, 10⁻⁶ M)
   7....treatment group (KMUP-1, 10⁻⁵ M)

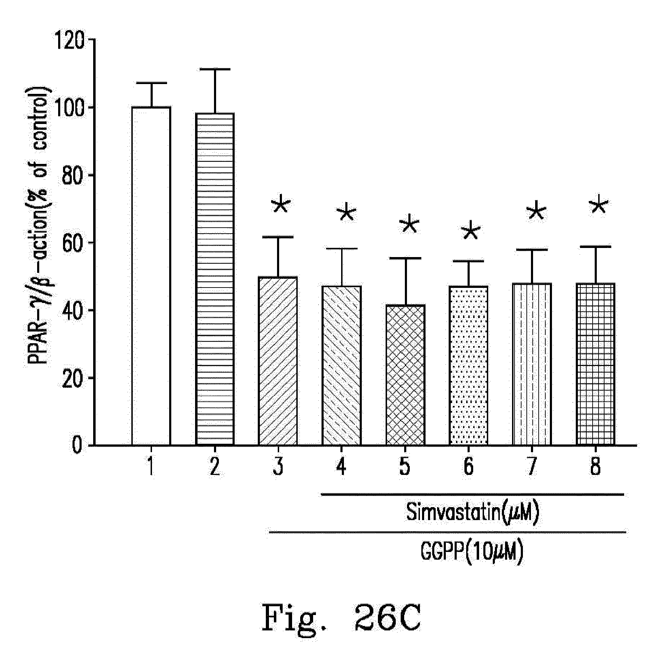

Fig. 26C shows the effects of simvastatin.
   1...control group 2...vehicle
   3...treatment group (simvastatin, positive control)
   4...treatment group (simvastatin, 10⁻⁹ M)
   5... treatment group (simvastatin, 10⁻⁸ M)
   6...treatment group (simvastatin, 10⁻⁷ M)
   7.... treatment group (simvastatin, 10⁻⁶ M)
   8.... treatment group (simvastatin, 10⁻⁵ M)

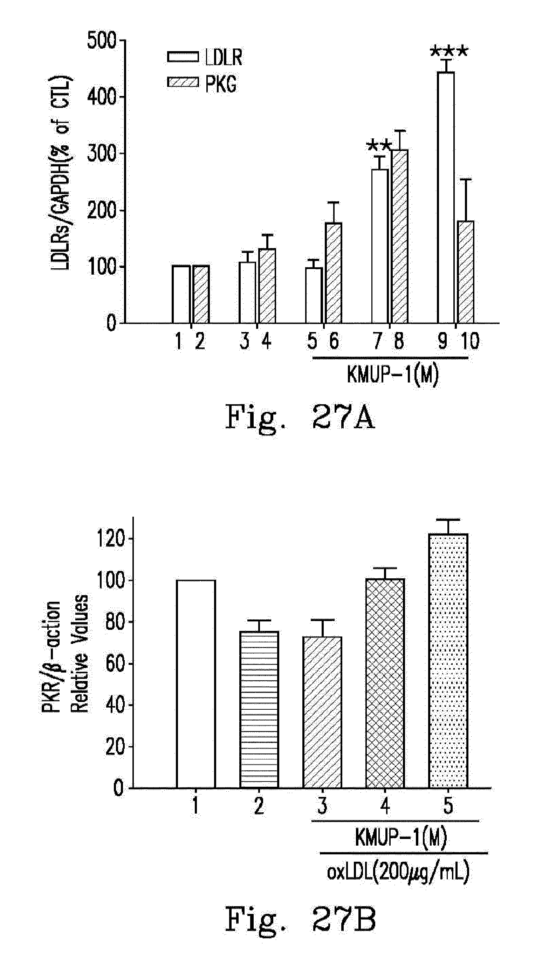

Figs. 27A-27B show the PKA protein expressions.
Fig. 27A shows the effects of KMUP-1, in the presence of LDL.
   1...control group of LDL 2...control group of PKG
   3...LDLRs of LDL 4...LDLRs of PKG
   5...LDLR (KMUP-1, 10 µM) 6...PKG (KMUP-1, 10 µM)
   7....LDLR (KMUP-1, 20 µM) 8....PKG (KMUP-1, 20 µM)
   9....LDLR (KMUP-1, 40 µM) 10....PKG (KMUP-1, 40 µM)
Fig. 27B shows the effects of KMUP-1 with reversed oxLDL.
   1...control group 2...oxLDL
   3...oxLDL (200µg/ml)+ KMUP-1 (1 µM)
   4...oxLDL (200µg/ml)+ KMUP-1 (10 µM)
   5...oxLDL (200µg/ml)+ KMUP-1 (100 µM)

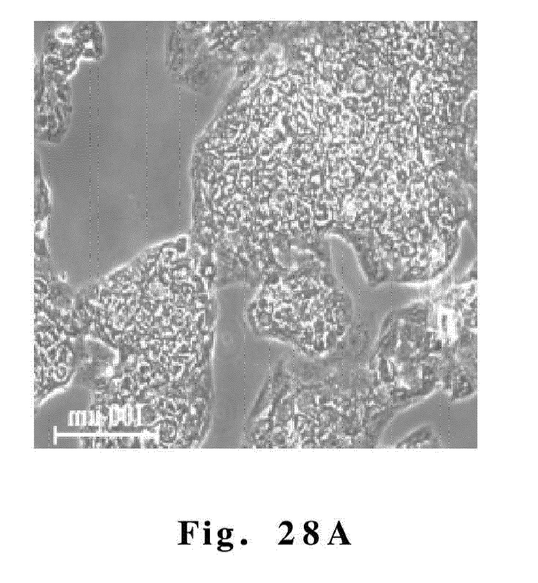

Figs. 28A-28B show the effects of KMUP-1 for the LDLRs.
Fig. 28A shows the bright field image of 1 x 10⁻⁴ M KMUP-1.

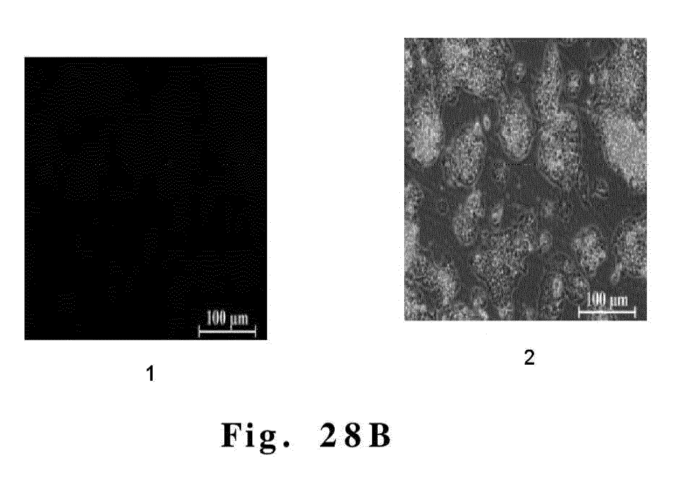

Fig. 28B shows the bright field and fluorescense staining image of the negative control.
   1...LDLR 2...bright field
Figs. 29A-29B show the effects of KMUP-1, simvastatin for the PKA.
Fig. 29A shows the bright field image of KMUP-1.

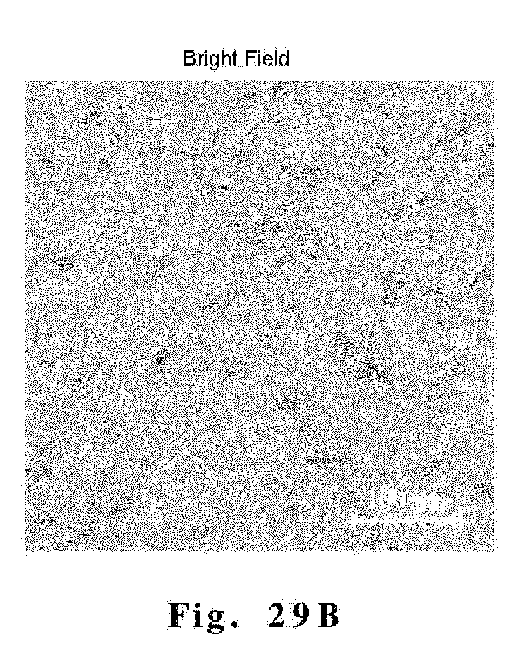

Fig. 29 B shows the bright field of simvastatin.

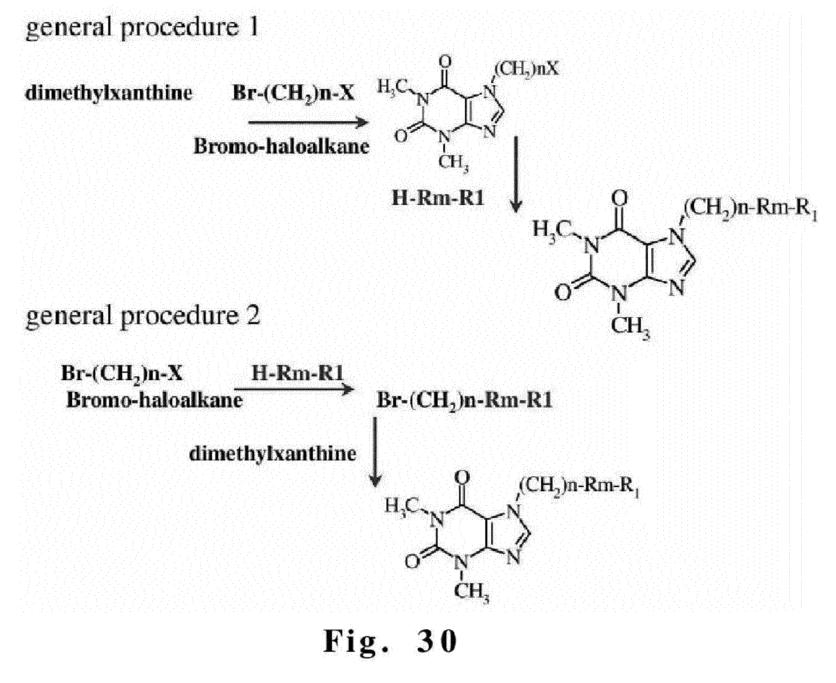

Fig. 30 shows the general procedures 1 and 2.

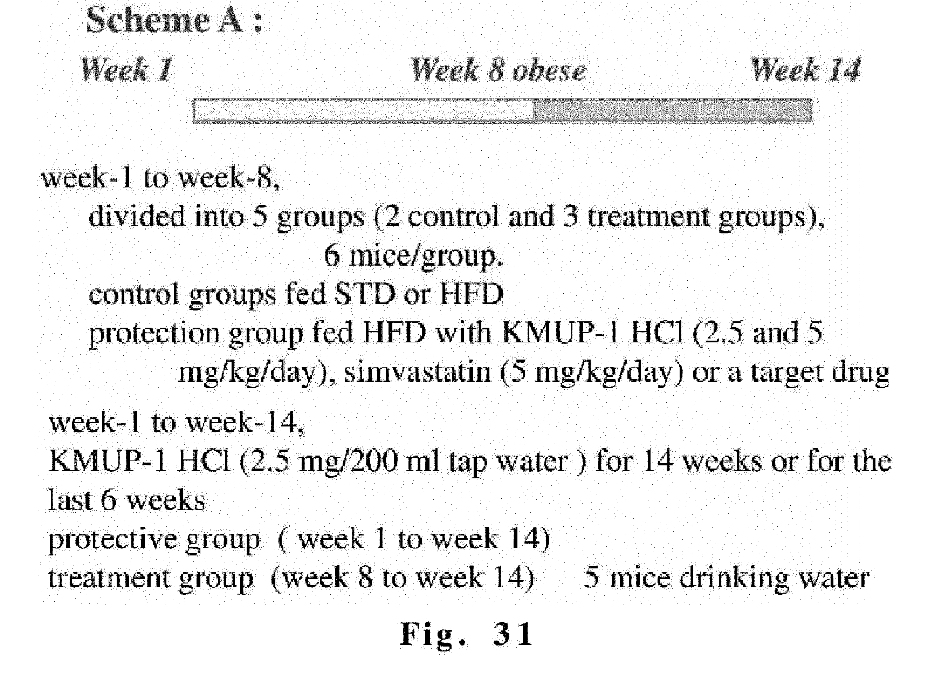

Fig. 31 shows the Scheme A of animal experiment model.

### DETAILED DESCRIPTION OF THE PREFERRED EMBODIMENT

The present invention will now be described more specifically with reference to the following embodiments. It is to be noted that the following descriptions of preferred embodiments of this invention are presented herein for the purposes of illustration and description only; they are not intended to be exhaustive or to be limited to the precise form disclosed.

In accordance with an aspect of the present invention, both Piperazinyl Analogs and Piperazinyl Complex Analogs are classified into two categories, in which Piperazinyl Analogs includes KMUPs Complex and Sildenafil Analogs Complex, and another Piperazinyl Complex Analogs includes KMUPs Complex and Sildenafil Analogs Complex. The RX group is a carboxylic group donator, which bonds to one of the active agents selected from a KMUPs derivative compounds and a Sildenafil Analogs, which can produce KMUPs Complex or Sildenafil Analogs Complex. KMUPs Complex can be designated by the general formula KMUPs-RX, and Sildenafil Analogs Complex is known as the general formula Sildenafil Analogs-RX.

In an embodiment of the present invention, a combination therapy is disclosed for treating and/or preventing liver disease. The compositions are formulated and administered in the manner detailed herein. The active agent of the KMUPs derivative compounds and Sildenafil Analogs compound may be effectively used alone or in combination with one or more active agents depending on the desired target therapy. Combination therapy includes administration of a single pharmaceutical dosage composition which contains one compound selected from a KMUPs derivative compound and a Sildenafil Analogs compound and one or more of the different active agents, as well as other administration types of different active agent, and each active agent has its own separate pharmaceutical dosage formulation.

When used in combination, in some embodiments, the compound of one selected from a KMUPs derivative compound and a Sildenafil Analogs compound may be administered as a single pharmaceutical dosage composition that contains different active agents. In other embodiments, separate dosage compositions are administered; the above mentioned compound and the other additional pharmaceutical agents may be administered at essentially the same time, for example, concurrently, or at separately staggered times, for example, sequentially. In certain examples, the individual components of the combination may be administered separately, at different times during the course of therapy, or concurrently, in divided or single combination forms. Also disclosed is, for example, simultaneous, staggered and alternating treatment.

For example, one compound selected from a KMUPs derivative compound, KMUPs-RX complex, Sildenafil Analogs and a Sildenafil Analogs-RX complex compound can be administered to the patient together in a single oral dosage composition such as a tablet or capsule, or each agent administered in separate oral dosage formulations. Where separate dosage formulations of different active agent are used, they can be administered in another administration type at essentially the same time. An example of combination treatment may be by any suitable administration route including oral (including buccal and sublingual), rectal, nasal, vaginal, and parenteral (including subcutaneous, intramuscular, intravenous and intradermal). Topical administrations include, but are not limited to, sprays, plaster, mist, aerosols, solutions, lotions, gels, creams, ointments, pastes, unguents, emulsions and suspensions, with oral or parenteral being preferred. The preferred route may vary with the condition and age of the recipient.

While it is possible for the administered ingredients to be administered alone, it is preferable to include them as part of a pharmaceutical formulation. The formulations of the present invention comprise the administered ingredients, as defined above, together with one or more acceptable carriers and optionally other additional therapeutic ingredients. The carrier(s) must be "acceptable" in the sense of being compatible with the other ingredients of the formulation and not deleterious to the recipient.

According to the above-mentioned aspect of the present invention, different active agent may include additional compounds according to the invention, or one or more other pharmaceutically active agents. In preferable embodiments, the inventive compositions will contain the active agents, including the inventive combination of therapeutic agents, in an amount effective to treat an indication of symptoms.

Preferably, in one embodiment, different active agents consist of Statin analogues, co-polymer, poly-γ-polyglutamic acid derivative, D-ascorbic acid, L-ascorbic acid, DL-ascorbic acid, oleic acid, phosphoric acid, citric acid, nicotinic acid and sodium carboxyl methylcellulose (sodium CMC).

The therapeutic substance being distributed is suitably administered in any way in which at least some (preferably about 1 wt. % to about 100 wt. %) of the substance reaches the bloodstream of the organism. Thus, the substance can be administered enterally (via the alimentary canal) or parenterally (via any route other than the alimentary canal, such as, e.g., through intravenous injection, subcutaneous injection, intramuscular injection, inhalation percutaneous application, etc.).

According to a further feature of this aspect of the invention there is disclosed a method for producing a treatment effect for liver disease in a warm-blooded animal, such as a man, goat, lamb, pig, cow, chicken, duck in need of such treatment which comprises administering to said animal an effective amount of Piperazinyl Analogs and Piperazinyl Complex Analogs, or a pharmaceutically acceptable salt, solvate, solvate of such a salt or a prodrug thereof.

A pharmaceutical composition is disclosed in the present application, which contains the active agent consisting of Piperazinyl Analogs and Piperazinyl Complex Analogs, in which the active agent is one selected from the group consisting of KMUPs compound, KMUPs-RX complex compound, Sildenafil Analogs derivative compound and Sildenafil Analogs-RX complex compound.

A pharmaceutical composition is disclosed in the present application, in which the active agent is a Piperazinyl moiety compound for treating a liver disease. A KMUPs derivative compound which can increase cyclic guanosine monophosphate (cGMP) to adipogenesis or lipoysis led us to reconsider the role of phosphorylated hormone-sensitive triglayceride lipase/Adipose triglayceride lipase (p-HSL/ATGL). KMUPs derivative, which is obtained by reacting a theophylline compound with a piperazine compound and then recrystallizing the intermediate therefrom, is disclosed in the present invention. The Piperazinyl Analogs and Piperazinyl Complex Analogs has the effect of treating a liver disease and the benefits of good solubility, low toxicity and safety.

Preferably, the pharmaceutical composition includes one of a KMUPs derivative compound having formula I or its salts, 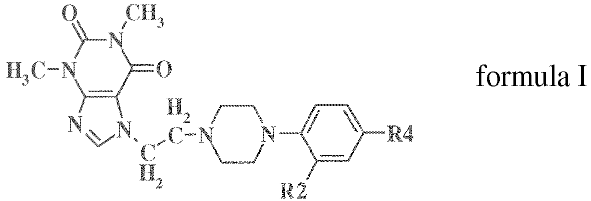 characterized in that R₂ and R₄ are each selected independently from the group consisting of a C1-C5 alkoxy group, hydrogen atom, nitro group, and a halogen atom. The above-mentioned halogen refers to fluorine atoms, chlorine atoms, bromine atoms and iodine atoms.

The term "KMUPs derivative compound " as used herein refers to one selected from the group consisting of KMUP-1, KMUP-2, KMUP-3, KMUP-4 and its pharmaceutical acceptable salts. Preferably, the pharmaceutical composition further includes at least one of a pharmaceutically acceptable carrier and an excipient.

Preferably, in one embodiment, the compound of formula I is KMUP-1, characterized in that R₂ is chlorine atom and R₄ is hydrogen atom, which has the general chemical name 7-[2-[4-(2-chlorophenyl)piperazinyl]ethyl]-1, 3-dimethyl xanthine. The compound of formula I is KMUP-2, characterized in that R₂ is a methoxy group and R₄ is hydrogen atom, which has the chemical name 7-[2-[4-(2-methoxybenzene)piperazinyl]ethyl]-1,3-dimethylxanthine. In another embodiment, the compound of formula I is also KMUP-3, characterized in that R₂ is hydrogen and R₄ is a nitro group, which has the chemical name 7-[2-[4-(4-nitrobenzene)piperazinyl]ethyl]-1,3-dimethylxanthine. In another embodiment, the compound of formula I is also KMUP-4, characterized in that R₂ is a nitro group and R₄ is hydrogen atom, which has the chemical name 7-[2-[4-(2-nitrobenzene)piperazinyl]ethyl]-1,3-dimethylxanthine.

The present invention discloses a KMUPs complex compound having a formula II of KMUPs-RX or formula III of KMUPs-RX-RX, 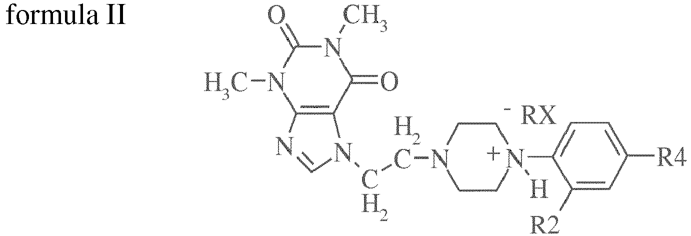 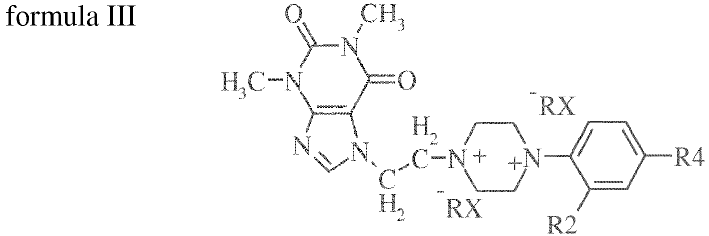
characterized in that R₂ and R₄ are each selected independently from the group consisting of a C1-C5 alkoxy group, hydrogen atom, nitro group, and a halogen atom;
RX includes a carboxylic group which is donated from one of a Statin analogues, co-polymer, poly-γ-polyglutamic acid derivative, D-ascorbic acid, L-ascorbic acid, DL-ascorbic acid, oleic acid, phosphoric acid, citric acid, nicotinic acid and sodium carboxymethyl cellulose (sodium CMC); and
⁻RX is an anion of a carboxylic group donated from one of statins analogues, co-polymer, poly-γ-polyglutamic acid derivative, D-ascorbic acid, L-ascorbic acid, DL-ascorbic acid, oleic acid, phosphoric acid, citric acid, nicotinic acid and sodium carboxymethyl cellulose (sodium CMC).

Preferably, the above statins analogues are commercially available statin derivative drugs, including Atorvastatin, Cerivastatin, Fluvastatin, Lovastatin, Mevastatin, Pravastatin, Rosuvastatin, Simvastatin and Pravastatin acid. A co-polymer is one selected from the group consisting of hyaluronic acid (HA), polyacrylic acid (PAA), polymethacrylates (PMMA), Eudragit, dextran sulfate, heparan sulfate, polylactic acid or polylactide (PLA), polylactic acid sodium (PLA sodium) and polyglycolic acid sodium (PGCA sodium). A poly-γ-polyglutamic acid (γ-PGA) derivative is one selected from the group consisting of sodium alginate, γ-polyglutamic acid (γ-PGA), sodium γ-polyglutamate (sodium γ-PGA), and alginate-poly-1-lysine-alginate (APA). Hyaluronic Acid is polymer, which is composed of alternating units of N-acetyl glucosamine (NAG) and D-glucuronic acid. Eudragit is a trade name for a series of copolymers derived from the esters of acrylic and methacrylate acid.

To achieve the purpose above, formula I salts, formula II and formula III can be synthetically produced from the 2-Chloroethyltheophylline compound and piperazine substituted compound. 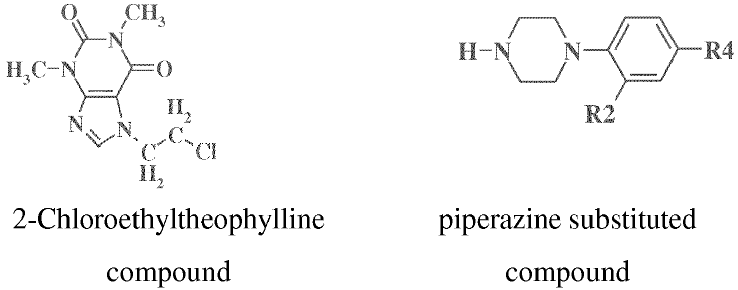 The compounds of formula I salts and KMUPs complex compound set forth in the examples below were prepared using the following general procedures.

General procedure 1 includes steps of dissolving 2-Chloroethyl theophylline and piperazine substituted compound in a hydrous ethanol solution, and the amount of reagent should be conjugated depending on the molecular weight percentage. After adding a strong base e.g. sodium hydroxide (NaOH) or sodium hydrogen carbonate (NaHCO₃) to make the solution more alkaline or more basic, a heating procedure was performed under reflux for three hours. Allowed to stand overnight, the cold supernatant was decanted solvents were efficiently removed by vacuum concentration, and then the residue was dissolved with a one-fold volume of ethanol and a three-fold volume of 2N hydrochloric acid (HCl), kept at 50°C to 60°C to make a saturated solution (pH 1.2). The saturated solution was sequentially treated, decolorized with activated charcoal, filtered, deposited overnight and filtered to obtain KMUP-1 HCl in the form of white crystals. As illustrated in general procedure 1, characterized in that the substitute group N-Rm-R1 is one selected from a group consisting of an azirine ring 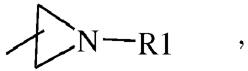 an azetidine ring 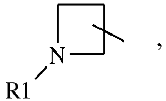 a pyrrolidine ring 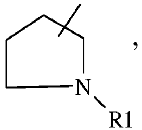 a piperidine ring 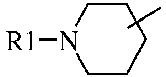 and a piperazinyl ring 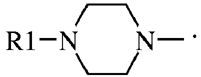 R1 is one selected from a group consisting of hydrogen, a halogen, a C1-C6 alkyl group and a C1-C5 alkoxyl group.

General procedure 2 includes steps of dissolving 2-Chloroethyl theophylline and piperazine substituted compound in a hydrous ethanol solution, and the amount of reagent should be conjugated depending on the molecular weight percentage. Then, heating procedure was performed under reflux for three hours. Allowed to stand overnight, the cold supernatant was decanted solvents were efficiently removed by vacuum concentration, and then the residue was dissolved with a one-fold volume of ethanol and a three-fold volume of 2N hydrochloric acid (HCl), kept at 50°C to 60°C to make a saturated solution (pH 1.2). The saturated solution was sequentially treated, decolorized with activated charcoal, filtered, deposited overnight and filtered to obtain KMUP-1 HCl with a white crystal.

According to general procedure 1 or 2, KMUPs derivatives of formula I can be synthetically produced directly from the 2-Chloroethyl-theophylline compound and piperazine substituted compound. Preferably, a theophylline-based moiety compound derivative, i.e. KMUPs derivative, which is obtained by re-acting a theophylline compound with a piperazine compound and then recrystallizing the intermediate therefrom, is disclosed in the present invention.

Preferably, the pharmaceutically acceptable salts of KMUP-1, KMUP-2, KMUP-3 and KMUP-4 are citric acid, nicotinic acid and hydrochloride.

Thereby, the KMUPs compound represents a KMUPs complex compound. Preferably, in one embodiment, KMUP-1 is dissolved in a mixture of ethanol and γ-Polyglutamic acid. The solution is reacted at a warmer temperature, methanol was added thereto under room temperature, and the solution is incubated overnight for crystallization and filtrated to obtain the KMUP-1-γ-Polyglutamic complex. According to the above-mentioned general procedure 1 or 2, a sufficient amount of KMUPs derivative reacts with a carboxyl group "RX" to form formula II of the KMUPs complex compound. Further, the term "RX" group can refer to the Statin analogues, co-polymer, poly-γ-polyglutamic acid derivative, D-ascorbic acid, L-ascorbic acid, DL-ascorbic acid, oleic acid, phosphoric acid, citric acid, nicotinic acid or sodium carboxymethyl cellulose (sodium CMC), which contains a sufficient amount of the carboxyl group and can react with the piperazine group of KMUPs derivative to prepare the above formula III of the KMUPs complex according to the method in the present invention. The synthesized KMUPs complex compound shows a pro-drug and multiple therapeutic functions in the body via a chemical or an enzymatic hydrolysis. In formula II, the KMUPs complex compound is also known as a KMUP-RX complex compound and formula III is also represented as a KMUP-RX-RX complex compound.

Specifically speaking, the KMUPs-RX complex compounds, in one embodiment, as various KMUPs derivatives, include a KMUP-1-Atorvastatin complex compound, KMUP-2-Atorvastatin complex compound, KMUP-3-Atorvastatin complex compound, KMUP-4-Atorvastatin complex compound, KMUP-1-Cerivastatin complex compound, KMUP-2-Cerivastatin complex compound, KMUP-3-Cerivastatin complex compound, KMUP-4-Cerivastatin complex; KMUP-1-Fluvastatin complex compound, KMUP-2-Fluvastatin complex compound, KMUP-3-Fluvastatin complex compound, KMUP-4-Fluvastatin complex compound; KMUP-1-Lovastatin complex compound, KMUP-2-Lovastatin complex compound, KMUP-3-Lovastatin complex compound, KMUP-4-Lovastatin complex; KMUP-1-Mevastatin complex compound, KMUP-2-Mevastatin complex compound, KMUP-3-Mevastatin complex compound, KMUP-4-Mevastatin complex compound, KMUP-1-Pravastatin complex compound, KMUP-2-Pravastatin complex compound, KMUP-3-Pravastatin complex compound, KMUP-4-Pravastatin complex compound, KMUP-1-Rosuvastatin complex compound, KMUP-2-Rosuvastatin complex compound, KMUP-3-Rosuvastatin complex compound, KMUP-4-Rosuvastatin complex compound, KMUP-1-Simvastatin complex compound, KMUP-2-Simvastatin complex compound, KMUP-3-Simvastatin complex compound, KMUP-4-Simvastatin complex; KMUP-1-ascorbic acid complex compound, KMUP-2-ascorbic acid complex compound, KMUP-3-ascorbic acid complex compound, KMUP-4-ascorbic acid complex; KMUP-1-phosphoric acid complex compound, KMUP-2-phosphoric acid complex compound, KMUP-3-phosphoric acid complex compound, KMUP-4-phosphoric acid complex; KMUP-1-CMC complex compound, KMUP-2-CMC complex compound, KMUP-3-CMC complex compound, KMUP-4-CMC complex compound, KMUP-1-hyaluronic complex compound, KMUP-2-hyaluronic complex compound, KMUP-3-hyaluronic complex compound, KMUP-4-hyaluronic complex compound, KMUP-1-polyacrylic complex compound, KMUP-2-polyacrylic complex compound, KMUP-3-polyacrylic complex compound, KMUP-4-polyacrylic complex compound, KMUP-1-Eudragit complex compound, KMUP-2-Eudragit complex compound, KMUP-3-Eudragit complex compound, KMUP-4-Eudragit complex; KMUP-1-polylactide complex compound, KMUP-2-polylactide complex compound, KMUP-3-polylactide complex compound, KMUP-4-polylactide complex; KMUP-1-polyglycolic complex compound, KMUP-2-polyglycolic complex compound, KMUP-3-polyglycolic complex compound, KMUP-4-polyglycolic complex compound, KMUP-1-dextran sulfate complex compound, KMUP-2-dextran sulfate complex compound, KMUP-3-dextran sulfate complex compound, KMUP-4-dextran sulfate complex compound, KMUP-1-heparan sulfate complex compound, KMUP-2-heparan sulfate complex compound, KMUP-3-heparan sulfate complex compound, KMUP-4-heparan sulfate complex compound, KMUP-1-alginate complex compound, KMUP-2-alginate complex compound, KMUP-3-alginate complex compound, KMUP-4-alginate complex compound, KMUP-1-γ-PGA complex compound, KMUP-2-γ-PGA complex compound, KMUP-3-γ-PGA complex compound, KMUP-4-γ-PGA complex compound, KMUP-1-APA complex compound, KMUP-2-APA complex compound, KMUP-3-APA complex compound, KMUP-4-APA complex compound, etc.

In accordance with a further aspect of the present invention, depending on the desired clinical use and effect, the adaptable administration method for the pharmaceutical composition consisting of the KMUPs derivative, Sildenafil Analogs compound and KMUPs complex, the Sildenafil Analogs complex includes one selected from the group consisting of an oral administration, an intravenous injection, subcutaneous injection, an intraperitoneal injection, an intramuscular injection and a sublingual administration.

Preferably, in another embodiment, the term "Sildenafil Analogs compounds" as used herein refers to one selected from the group consisting of, Sildenafil, Hydroxyhomosildenafil, Desmethylsildenafil, Acetidenafil, Udenafil, Vardenafil and Homosildenafil. The compound Sildenafil has the chemical name 5-[2-etthoxy-5-(4-methylpiperazin-1-yl-sulphonyl)phenyl]-1-methyl-3-n-propyl-1,6-dihydro-7H-pyrazolo[4,3d]pyrimidin-7-one, or 1-[[3-(4,7-dihydro-1-methyl-7-oxo-3-propyl-1H-pyrazolo[4,3-d]pyrimidin-5-yl)-4-ethoxyphenyl]sulfonyl]-4-methyl-piperazine. The compound Hydroxyhomo-Sildenafil has the chemical name 1-[[3-(6,7-dihydro-1-methyl-7-oxo-3-propyl-1H-pyrazolo[4,3-d]pyrimidin-5-yl)-4-ethoxy-phenyl] sulfonyl]-4-hydroxyethyl-piperazine. The compound Desmethyl-Sildenafil has the chemical name 5-[2-ethoxy-5-(1-piperazinylsulfonyl)-phenyl]-1,6-dihydro-1-methyl-3-propyl-7H-pyrazolo-[4,3d]-pyrimidin-7-one. The compound Acetidenafil has the chemical name 5-{2-ethoxy-5-[2-(4-ethyl-piperazine-1-yl)-acetyl]phenyl}-1-methyl-3-n-propyl-1,6-dihydro-7H-pyrazolo[4,3-d]pyrimidin-7-one. The compound Udenafil has the chemical name 5-[2-propyloxy-5-(1-methyl-2-pyrollidinylethylamidosulfonyl)phenyl] -1-methyl-3-propyl-1,6-dihydro-7H-pyrazolo[4,3-d]pyrimidine-7-one. The compound Vardenafil has the chemical name 2-[2-ethoxy-5-(4-ethyl-piperazin-1-yl-sulfonyl)phenyl]-5-methyl-7-propyl-1H-imidazo[5,1-f][1,2,4] triazin-4(3H)-one. The compound HomoSildenafil has the chemical name 5-[2-ethoxy-5-[(4-ethyl-1-piperazinyl)sulfonylphenyl]-1,6-dihydro-1-methyl-3-propyl-7H-pyrazolo[4,3-d]pyrimidin-7-one.

Sildenafil Analogs derivative compounds compound

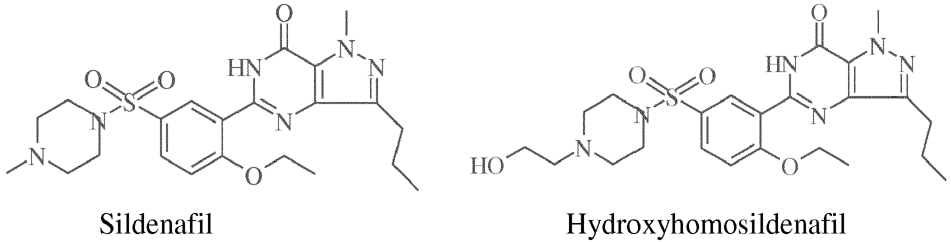

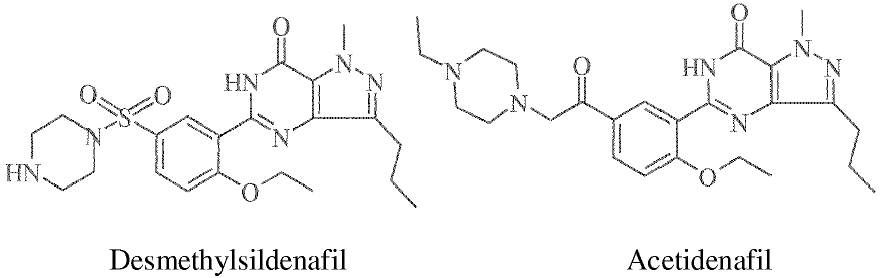

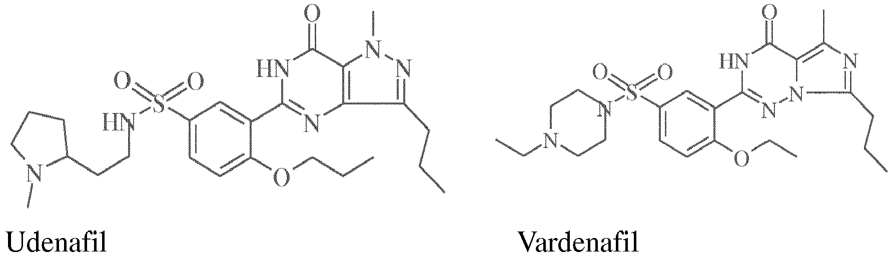

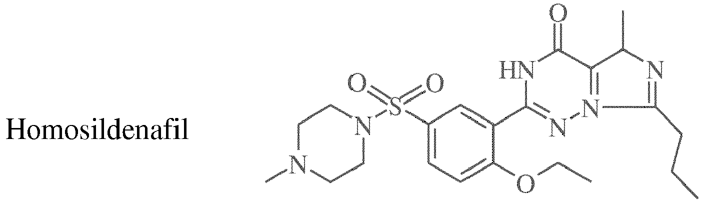

Preparation of Sildenafil analogs-RX complex compounds from Sildenafil citrate and Sildenafil

Sildenafil analogs complex compound, also designated by the general formula Sildenafil Analogs-RX complex compound, were prepared according to the abovementioned general procedure. Preferably, in an embodiment, crude Sildenafil citrate is blended and suspended in a sodium hydroxide solution to dissolve the components. After filtration of the sodium citrate, the precipitated Sildenafil base is added to an equal molecular-weight of ascorbic acid which was dissolved in methanol to react at 50°C. After overnight cooling in a glass flask, a white precipitate was obtained by filtration and then re-crystallized as Sildenafil ascorbate from ethanol.

In another embodiment, Sildenafil citrate was dissolved in diluted water, adjusted with an HCl solution to pH 7.0 and separated into an ethyl acetate fraction to remove the citric acid, hydrochloride and a sodium chloride into a water fraction. The obtained Sildenafil base in ethyl acetate was dried under a de-pressurized condition. Then one carboxylic group of the RX and Sildenafil base was dissolved in methanol to react at 50°C. After sitting overnight, white precipitate was obtained by filtration and then recrystallized as Sildenafil complex compounds from ethanol.

In another embodiment, Sildenafil HCl and sodium carboxylate of the RX were dissolved in methanol at an equal molecular weight to react at 50°C. After overnight cooling, a white precipitate was obtained by filtration and then recrystallized as Sildenafil complex compounds from ethanol. However, based on the abovementioned procedure, Sildenafil analogs-RX complex compounds can be prepared selectively with one of the hydrochloride salts of Sildenafil analogs derivatives and one of the RX group.

The term excipients or "pharmaceutically acceptable carriers or excipients" and "bio-available carriers or excipients" mentioned above include any appropriate compounds known to be used for preparing the dosage form, such as a solvent, dispersing agent, coating, anti-bacterial or anti-fungal agent, preservation agents or a delayed absorbent. Typically, such a carrier or excipient does not itself have therapeutic activity. Each formulation prepared by combining the derivatives disclosed in the present invention and the pharmaceutically acceptable carriers or excipients will not cause any undesired effects, allergy or other inappropriate effects when being administered to an animal or human. Accordingly, the derivatives disclosed in the present invention in combination with the pharmaceutically acceptable carrier or excipients are adaptable for clinical usage and in humans.

A therapeutic effect can be achieved by using suitable dosage forms, in part, depending on the use or the route of administration, e.g. venous, oral, and inhalation routes or via the nasal, rectal and vaginal routes. Such dosage forms should allow the compounds to reach target cells. About 0.1 mg to 1000 mg per day of the active ingredient is administered for patients with various diseases. Preferably, in one embodiment, a single oral dose of KMUPs derivative, Sildenafil Analogs derivative or KMUPs-RX complex, Sildenafil Analogs-RX complex compound is about 1-2.5 milligram per kilogram of body weight.

The carrier varies with each formulation, and the sterile injection composition can be dissolved or suspended in non-toxic intravenous injection diluents or a solvent such as 1, 3-butanediol. Among these carriers, the acceptable carrier may be mannitol or water. In addition, fixing oil and synthetic glycerol ester or di-glycerol ester are commonly used solvents. A fatty acid such as oleic acid, olive oil or castor oil and glycerol ester derivatives thereof, especially the oxy-acetylated type, may serve as the oil for preparing the injection and as a natural pharmaceutically acceptable oil. Such an oil solution or suspension may include the long chain alcohol diluents or dispersing agents, carboxylmethyl cellulose or an analogous dispersing agent. Other carriers are common surfactants such as Tween and Spans or another analogous emulsion, or a pharmaceutically acceptable solid, liquid or other bio-avaliable enhancement agent used for developing a formulation that is used in the pharmaceutical industry.

The composition for oral administration may use any acceptable oral formulation, which includes a capsule, tablet, pill, emulsion, aqueous suspension, dispersing agent or solvent. A carrier is generally used in an oral formulation. Taking a tablet as an example, the carrier may be lactose, corn starch and lubricant, and magnesium stearate is the basic additive. The diluents used in the capsule include lactose and dried corn starch. To prepare the aqueous suspension or the emulsion formulation, the active ingredient is suspended or dissolved in an oil interface in combination with the emulsion or the suspending agent, and an appropriate amount of a sweetening agent, flavor or pigment is added as needed.

The compound of the present invention can also be administered intravenously, as well as subcutaneously, parentally, into muscles, or by intra-articular, intracranial, intra-articular fluid and intra-spinal injections, aortic injection, sterna injection, intra-lesion injection or other appropriate administration.

The term "food compositions" mean products and ingredients, taken by mouth, the constituents of which are active in and/or absorbed by the gastrointestinal (G.I.) tract with the purposes of nourishment of the body and its tissues, refreshment and indulgence. Examples of food and beverage products are tea, ice cream, frozen fruits and vegetables, snacks including diet foods and beverages; meal substitute and meal replacements. Food compositions may bring any of the following benefits: healthy metabolism; life span extension; optimal growth and development of G.I. tract function; and avoidance of liver disease.

The present invention discloses a pharmaceutical composition in which the active agent is a Piperazinyl moiety compound for inhibiting lipid accumulation/mobilization from adipose tissues to the liver, reduces steatohepatitis and leads to lean body of fat pads, and is related to lowering body-weight via fat loss to protect from steatohepatitis in the liver.

Lipolysis in brown adipose tissue is a biochemical pathway responsible for the hydrolysis of triacylglycerol (TAG) stored in the oil globulets of adipose tissues. The hydrolysis of triacylglycerol generates non-esterified fatty acids, which are used as energy substrates in brown cell type adipose tissues. Enhancing protein kinase A (PKA) using caffeine can phosphorylate perilipin on oil globulets, resulting in lipolysis by hormone-sensitive triglayceride lipase (HSL)/p-HSL and adipose triglyceride lipase (ATGL).

G-protein-coupled receptors are the most common potential pharmacological targets but few reports indicate that GPCRs antagonists inhibit the adipogenesis of adipose tissues. GPCRs-mediated activation of major kinases, including mitogen-activated protein kinase (MAPK), extracellular signal-regulated protein kinases (ERK) and Mitogen-activated protein kinase kinase 1 (MEK1), is likely to become important for the identification of GPCR antagonists targeting adipogenesis or lipolysis.

Hepatic steaotosis may be induced by hyperadiposity via a HFD; HFD-induced hyperadiposity in the liver involves lipid accumulation combined with inflammation, which may lead to further liver damage. Steatohepatitis is usually accompanied by oxidative stress via reactive oxygen species (ROS) after long-term administration of a HFD. HFD-induced obesity causes oxidative stress via ROS, indirectly increasing MAPK and ERK expression via hepatocytes. We sought to suppress ROS and inhibit inflammatory tumor necrosis factor alpha (TNF-α) and Matrix metallopeptidase (MMP-9) in adipocytes surrounded by macrophages.

Adipose tissue inflammation in obesity is characterized by macrophage types affected by classically activated macrophages (M1, CAM)/ alternatively activated macrophages (M2, AAMs) switching; and immunostaining for macrophages was massive in most HFD-induced liver inflammation.

Therapies to raise the levels of high-density lipoprotein (HDL) and lower low-density of lipoprotein (LDL) levels are thought to exert atheroprotective effects via activation of eNOS, elevation of the peroxisome proliferator activated receptor γ (PPARγ) and inhibition of the scavenger receptor class B type I (SR-B1).

Application of mevalonate to liver cells results in biosynthesis of the isoprenoid compounds farnesyl pyrophosphate (FPP) and geranylgeranyl pyrophosphate (GGPP), levels of which are reduced by HMG CoA reductase inhibitor statins; GGPP-derived activation of RhoA/ROCK II and the following upregulation of PPARγ are involved in increasing high density lipoprotein (HDL). ATP-binding cassette transporter ABCA1 (member 1 of human transporter sub-family ABCA), and apolipoprotein A-I (ApoA-I) are involved in the regulation of cholesterol efflux and are the major protein components of HDL.

Cellular cholesterol homeostasis is accomplished, in part, by PPARs and Liver X receptor (LXRα). Statin-induced inactivation of RhoA/ROCK contributes to activation of LXRα/PPARs and their pleiotropic effects. Isoprenoid intermediates affect PPARs and LXRα activation. Activation of isoprenoid produces FPP and GGPP, which inhibit ABCA1 directly through antagonism of LXRα and indirectly through RhoA by activation of geranylgeranylation. PPARγ is expressed in fat storage and associated inflammation.

Effects on weight gain, food intake and lipid profiles in serum

Table 1 shows the 8-week body-weight gain of animals fed with a standard diet (STD) or a high-fat diet (HFD). Consumption of HFD for 8 weeks significantly increased body-weight gain compared to the STD group (p < 0.05). KMUP-1 HCl (2.5, 5mg/kg p.o.) and simvastatin supplementation (5 mg/kg p.o.) reduced body-weight gain, compared to the control HFD group (p<0.05). The reduction of body-weight gain and remained Triacylglycerol (TG) by simvastatin, was less and higher than that of KMUP-1, respectively.

HFD caused dramatic increases in serum TG, total cholesterol and LDL cholesterol compared with the STD group. HFD-induced hypercholesterolemia was significantly improved by KMUP-1 supplementation. Particularly, the HDL cholesterol level was significantly increased by KMUP-1 and simvastatin. When the food intake in animals fed a HFD after maturity (8 weeks) slowed down, the period of feeding was prolonged to 14 weeks. Some factors that affected the food intake by the animals remained unclear.

Table 1 shows effects of KMUP-1 and simvastatin on lipid levels, body weight and food intake of mice fed with HFD for 8 weeks.

**Table 1**

| | STD | HFD | HFD+ target drug (mg/kg) | | |
|---|---|---|---|---|---|
| | | | KMUP-1 | | Simva |
| | | | 2.5mg/kg | 5mg/kg | 5mg/kg |
| TG in serum | 107.2±6.1 | 166.8±5.3 | 74.5±5.1** | 72.7±4.7** | 82.7±6.3** |
| TG of liver | 42.3±3.2 | 78.6±5.3^{##} | 28.4±3.6** | 26.12±3.1** | 35.5±2.** |
| Tot. chol. | 78.7±1.9 | 206.8±13.4^{##} | 133.0±5.1* | 125.5±9.8* | 133.7±4.3* |
| HDL | 60.4±1.6 | 68.4±3.5^{#} | 103.6±4.2* | 118.3±5.7* | 103.2±2.5* |
| LDL | 6.0±0.3 | 31.6±7.0^{##} | 14.2±1.4* | 14.2±2.2* | 15.3±1.3* |
| intake | 4.0±0.2 | 2.4±0.1^{#} | 2.3±0.1* | 2.2±0.1* | 2.1±0.1* |
| initial BW | 21.1±0.3 | 22.1±0.8 | 22.0±0.3 | 21.2±0.7 | 21.1±0.8 |
| final BW | 24.1±0.5 | 29.1±0.9^{#} | 25.7±0.7* | 24.3±0.5* | 23.9±0.9* |
| BW gain | 3.0±0.4 | 6.9±0.7^{#} | 3.7±0.5* | 3.1±0.6*± | 2.8±0.3* |

| | | | | | |
|---|---|---|---|---|---|
| (note) STD = standard diet, HFD= high-fat diet, Simva = Simvastatin, TG=Triacylglycerol (mg/dl), chol.=cholesterol (mg/dl), Tot. chol.= total cholesterol (mg/dl), HDL = HDL cholesterol (mg/dl), LDL= LDL cholesterol (mg/dl), intake =food intake (g/day), initial BW = initial Body weight (g), final BW =Body weight (g), BW gain = Body weight gain (g/day), | | | | | |

Table 2 shows effects of KMUP-1-RX on lipid levels, of mice.

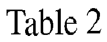

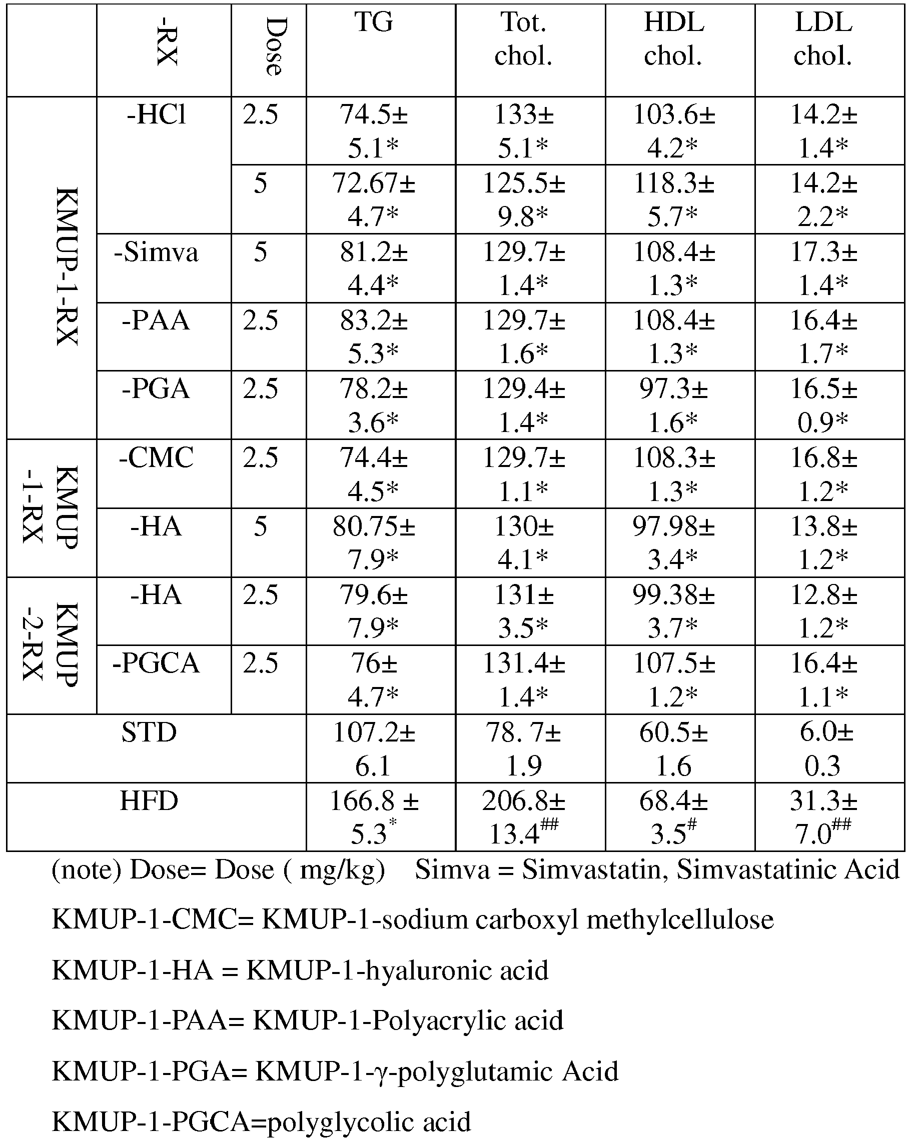

### Weight changes and gross morphology of the liver:

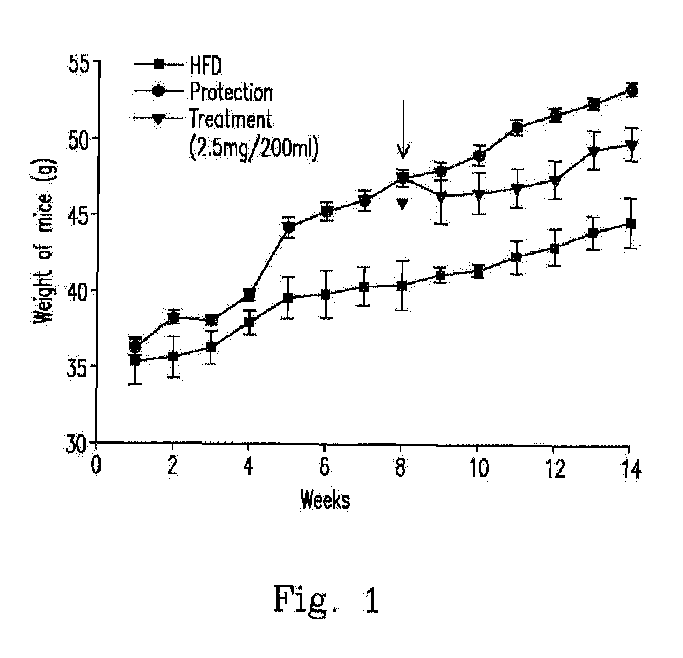

Fig.1A showed that drinking KMUP-1 HCl (2.5 mg/200 ml water) by mice fed a HFD decreased the body weight in both the protection and treatment groups. Fatty tissues were characteristically found on the surface of liver fed with HFD (Fig. 2A). Fatty liver was markedly decreased in the protective group and this effect was more prominent than in the treatment group. (Fig. 2C).

### HFD-induced SR-B1 expression in the liver:

Exploring KMUP-1 on increased HDL, drinking KMUP-1 was observed to inhibit HFD-induced hepatic SR-B1 expression (Fig. 3A) and promote PKA/PKG expression in both the protection and treatment groups (Fig. 3B).

### Measurement of oil globulets in liver:

10, 25, 50 and 100 µm standards are shown at the bottom of Fig. 4A. The estimated diameter of each oil globulet was 6, 21, 24 and 25 µm. Fig. 4B shows the variation of diameter changes and number of oil globulets. The counting of oil globulets was performed from larger to smaller ones until the observation reached its limit.

### H&E staining of liver:

Fig. 5A shows the gross morphology of inflammatory liver isolated from a fatty mouse liver fed with HFD for 14 weeks, which was rich in white fat tissues compared to the red-brown liver found in the protection group supplemented with KMUP-1+HFD for 14 weeks (Fig. 5B).

Fig. 5 shows Hematoxylin and eosin (H&E) staining of liver sections in which macrovesicular fat globules (white arrow) cleaned with organic solvent were found in liver of mice treated with HFD for 14 weeks/last 6 weeks in the treatment or protection group (Fig. 5B and 5C). Obese mice supplemented with HFD at week 8 were treated with oral KMUP-1 (2.5 mg/kg) for 6 weeks from week 8 to week 14. Mallory's hyaline bodies and fat globules were found (Fig. 5B). Pretreatment with oral KMUP-1 (2.5 mg/kg) during the last 6 weeks/14 weeks reduced fat globules and Mallory's hyaline bodies, which almost disappeared (Fig. 5C).

### IHC staining of inflammatory TNF-α and MMP-9 in steatohepatitis:

Fig. 6 shows administration of KMUP-1 (2.5 mg/kg/day) for 14 weeks (protection group, Fig. 6B) and 6 weeks (treatment group, Fig. 6C), which inhibited partial TNF-α immunohistochemistry (IHC) on fatty liver slices of HFD mice.

The deep brown color of the antibody response to TNF-α were shaded in both the treatment and protection groups, in which the oil globulets almost disappeared, but the color response could not be reduced substantially, indicating an incomplete anti-inflammatory effect (Fig. 7A). The deep brown color of the anti-body response to MMP-9 was also shaded by the treatment and almost disappeared in the protection group (Fig. 7B). The oil globulet number and changes of oil globulet diameter were sharply reduced in the treatment and protection groups. Even in the negative control, without the addition of an antibody, the oil globulets in the HFD group decreased or disappeared after KMUP-1 treatment.

### IHC staining of HSL/p-HSL/ATGL in steatohepatitis:

In Figs. 7C and 7D, the IHC of HSL and p-HSL in a HFD control are dark brown, indicating large amounts of inflammatory proteins or enzymes, including inflammatory MMP-9/TNF-α, inactive HSL/p-HSL/ATGL, hyperadiposity via multiple white oil globulets and ROS production in an obese liver. In the treatment and the protection groups, decreased white oil globulets indicated that lipolytic activity resulted from the activation of p-HSL but not the total form HSL by KMUP-1. HSL partly increased after treatment or protection with KMUP-1 but was also partly changed into p-HSL accompanied by active ATGL. The accompanying changes in the oil globule number and diameter by HSL are illustrated in Fig. 7E. Antibody responses, accompanied by a multiple oil globulet number, to p-HSL staining, were more prominent in the protection group than the treatment groups, indicating the major role of p-HSL compared to HSL in inhibiting steatohepatitis by KMUP-1.

Likewise, antibody responses and oil globulet number changes shown by ATGL staining were more prominent in the protection group than the treatment groups, indicating the important role of ATGL in lipolysis induced by KMUP-1 to inhibit steatohepatitis (Fig. 7E).

### IHC staining of type 1 or type 2 macrophages (M1 or M2) in steatohepatitis:

Fig. 8 and Fig. 9 indicate by IHC increases and decreased in M1 or M2 macrophages, with F4/80 and CD11c dye (Fig. 8) for the M1 type and CD206/CD209a for M2 type macrophages. CD209a (Fig. 9) staining was the most sensitive for the antibody responses. Multiple white oil globulets were found in all HFD-induced liver, indicating hyperadiposity resulting in steatohepatitis. These white oil globulets also interefered with our observation of the color background to separate the contrast difference. The oil globulet number and diameter of M1 type macrophages are illustrated in Figs. 10A and 10B. The KMUP-1 protection group was shown in the right side and the treatment group was shown in the middle of these cytology figures. IHC images were obtained under the same light intensities by light microscopy for all specimens. On average, all anti-body responses to M1 or M2 type macrophages shared a class 4 saturation of color intensity, as calculated by computer. The oil globulet diameter changes in the protection and the treatment groups were dramatically different from the HFD control as shown in Figs. 10A, 10B, 11A and 11B.

### H&E staining of fat pads:

HFD-induced hypertrophy/hyperplasia of the white cell type epididymal fat pads, imaged by H&E staining, was inhibited by protection with KMUP-1 (2.5 mg/kg). Fig. 12 shows that the increased epididymal fat pad weight of mice fed with a HFD was reduced by treatment with KMUP-1 for 14 weeks. KMUP-1 (2.5 mg/kg/day) for 14 weeks significantly reduced the fat pad weight and fat cell diameter during the protection period (Fig. 13).

### IHC staining of eNOS/HSL and IL-10/TNF-α in epididymal fat pads:

Figs. 14, 15, 16, 17 show that HFD for 14 weeks induced IHC staining of eNOS, HSL, IL-10 and TNF-α present on the rim of adipocytes (black arrow). Protection with KMUP-1 (2.5 mg/kg/day) inhibited the IHC staining of eNOS/TNF-α and increased HSL/IL-10, resulting in the changes of immunoresponse of fat pad cells, respectively.

### HFD-induced HMG CoA reductase, ROCK II, PPARγ and ABCA1 expression in liver:

Oral KMUP-1 and simvastatin by gavage affected HMG CoA reductase (HMGR) expression in mice fed with HFD for 8 weeks. Mice fed a HFD showed downregulated HMG CoA reductase expression compared to STD. Both KMUP-1 HCl (2.5, 5 mg/kg) and simvastatin (5 mg/kg) significantly reversed HFD-induced downregulation of HMG CoA reductase expression in liver (Figs.18, 19). Additionally, KMUP-1 also activated PPARγ (Fig. 20) and ABCA1 (Fig. 21) and inactivated ROCK II in animals treated with HFD.

### Serum/vehicle and mevalonate-induced HMG CoA reductase expression:

In HepG2 cells supplemented with serum/vehicle-containing medium, expression was concentration-dependently increased by incubation with KMUP-1 or simvastatin (10⁻⁹-10⁻⁵M) for 24 hours (Figs. 22A, 22B). Application of mevalonate (60, 80, 100 µM) in HepG2 cells concentration-dependently reduced the expression of HMG CoA reductase (Fig. 23A). Mevalonate at 100 µM sharply inhibited HMG CoA reductase expression and this effect was prevented by adding KMUP-1 or simvastatin (10⁻⁵M), indicating the end-product feedback regulation phenomenon of HMG CoA reductase (Fig. 23B).

### RhoA/ROCK II inactivation and eNOS-enhancement:

KMUP-1 concentration-dependently inhibited the translocation of RhoA from cytosol to membranes in the HepG2 cells. ROCK II is the downstream effector of RhoA in hepatic cellular signaling. KMUP-1 or simvastatin (10⁻⁹-10⁻⁵ M) concentration-dependently reduced ROCK II protein expression due to the inhibition of RhoA translocation. KMUP-1 concentration-dependently increased the expression of eNOS, and accordingly resulted in RhoA/ROCK II inactivation in HepG2 cells.

### Increased PPARγ/ABCA1/ApoA-I/LXRα

KMUP-1 and simvastatin (10⁻⁹-10⁻⁵ M) increased the expression of PPARγ and ABCA1 in HepG2, suggesting that they could affect the lipid metabolism toward formation of HDL. KMUP-1 increased PPARγ □ by 2-3 fold which was consistent with the decreasing weight gain, indicating that the PPARγ activity of KMUP-1 could be involved in the reduction of body weight (Table 1). Both KMUP-1 and simvastatin (10⁻⁹-10⁻⁵ M) concentration-dependently increased ApoA-I and LXRα expression in HepG2 cells.

### cGMP-pathway and RhoA/ROCK II:

Both RhoA antagonist C3 exoenzyme (5 µg/ml) and ROCK antagonist Y27632 (10 µM) reduced ROCK II expression (Fig. 24A), which was increased by 10µM cGMP antagonist Rp-8-pCPT-cGMPS (8-(p-chloro-phenylthio)guanosine-3,5-monophosphorothioate) and inhibited by the combination with KMUP-1 (10 µM), indicating the involvement of a cGMP-pathway in HepG2 cells (Fig. 24B). In addition, KMUP-1, simvastatin, C3 exoenzyme and Y27632 all increased PPARγ (Fig. 24C) and ABCA1 expression (Fig. 24D). KMUP-1 also increased PPARγ □ in parallel with the decreased weight gain, indicating that PPARγ has an impotant role in decreasing weight gain (Fig. 24C; Table 1).

### RhoA/ROCK II activation in the presence of GGPP and FPP:

Application of exogenous GGPP (Fig. 25A) and FPP (Fig. 25B) increased RhoA/ROCK II expression, and KMUP-1 (10⁻⁹-10⁻⁵ M) attenuated this phenomenon in HepG2 cells. In contrast, simvastatin did not inactivate ROCK II in the presence of exogenous GGPP and FPP (Fig. 25C).

### Exogenous GGPP or FPP decreases PPARγ and ABCA1:

Incubation of HepG2 cells with FPP or GGPP (10 µM) alone suppressed the expression of PPARγ and ABCA1. Incubation of FPP (Fig. 26A) or GGPP (Fig. 26B) with KMUP-1 (10⁻⁹-10⁻⁵ M) reversed the expression of PPARγ and ABCA1, but simvastatin (Fig. 26C ) did not affect PPARγ in the presence of GGPP (10 µM).

### Biosynthesis of [¹⁴C]mevalonate:

KMUP-1 (10 µM) could not reduce [¹⁴C]mevalonate formation. In contrast, simvastatin inhibited [¹⁴C]mevalonate formation by 86.6±4.2%, compared to the vehicle group. [¹⁴C]HMG CoA reductase was examined to be >90% in purity and 20 units/mg in activity from (Sigma-Adrich, St. Louis, Mo, U.S.A) was used to determine [¹⁴C]mevalonate formation.

### IHC of LDLRs and expression of PKG/PKA:

HFD-induced LDLRs expression in the liver was estimated using IHC staining methods. Notably, drinking KMUP-1 HCl increased the hepatic LDLRs of animals fed with HFD in both the protection and treatment groups. Western blotting of LDLRs and PKA/PKG showed that KMUP-1 (10, 20, 40 M) could not significantly affect PKA protein expression in HepG2 cells in the presence of LDL (500µg/mL), a pathologic model of hyperlipidemia, but increased the expression of PKG (Fig. 27A). Howover, KMUP-1 (1, 10, 100 µM) reversed the 200 µg/mL oxidized low density lipoprotein, oxidized LDL (oxLDL)-induced reduction of PKA expression (Fig. 27 B).

### Fluoroscent staining of cellular LDLRs/PKA/HSL:

HepG2 cells were stained with fluorescence and treated with different concentrations of KMUP-1 or simvastatin for 24 hours. Ours results showed increased LDLR (green fluorescence) expression in HepG2 cells with different concentrations of KMUP-1 (10⁻⁶, 10⁻⁵, 10⁻⁴ M) or simvastatin (10⁻⁵ M). The expression intensity of HepG2 cells treated with KMUP-1 was compared to the control. KMUP-1 at concentrations >10⁻⁴ M, for unknown reasons, showed a decline in fluoresence, suggesting that such a concentration could be near the viability range of HepG2 cells (Figs. 28A, 28B). PKA and HSL (green fluorescence) also showed increased immunoreactivities in HepG2 cells treated with KMUP-1(Fig. 29A) or simvastatin (Fig. 29B). However, PKG immunoreactivity was not significantly affected by KMUP-1 (data not shown).

### Table 3 shows PPARγ/eNOS/Rho-kinase/p-HSL.

**Table 3**

| | PPAR-γ | eNOS | Rho-kinase | p-HSL |
|---|---|---|---|---|
| control group | 100 (%) | 100 (%) | 100 (%) | 100 (%) |
| KMUP-1 (0.1µM) | 123.5±17.3 | 121.6±16.5 | 82.8±7.8 | 120.7±15.3 |
| KMUP-1 (1.0µM) | 135.6±14.5 | 132.7±15.2 | 78.4±6.9 | 138.6±14.7 |
| KMUP-1 (10µM) | 142.6±15.4 | 138.9±15.2 | 75.8±6.4 | 144.6±12.3 |
| KMUP-2 (10µM) | 135.7±11.7 | 129.8±10.8 | 81.9±8.2 | 135.6±14.1 |
| KMUP-3 (10µM) | 140.8±15.7 | 131.5±13.7 | 79.4±6.9 | 138.2±14.4 |
| KMUP-4 (10µM) | 143.7±11.3 | 125.6±13.6 | 83.7±7.5 | 136.5±12.6 |

Table 4 shows eNOS, PDE-5A, ROCKII.

**Table 4**

| | eNOS | PDE-5A | ROCKII |
|---|---|---|---|
| control group | 100 (%) | 100 (%) | 100 (%) |
| KMUP-1 HCl | 155.16±14.8 | 64.5±4.5 | 42.5±6.3 |
| KMUP-1-CMC | 152.14±6.3 | 58.7±3.5 | 38.6±2.7 |
| KMUP-2 HCl | 147.21±8.6 | 68.8±5.3 | 40.8±3.7 |
| KMUP-3 HCl | 148.31±7.5 | 63.6±5.2 | 44.5±2.9 |
| KMUP-4 HCl | 135.24±7.5 | 73.4±3.4 | 37.6±2.8 |
| KMUP-1-Poly -glutamic acid | 131.50±6.7 | 78.2±3.6 | 56.6±3.4 |
| KMUP-1-alginic acid | 155.16±14.8 | 64.5±4.5 | 42.5±6.3 |

KMUP-1 improved high-fat-diet (HFD)-induced dyslipidemia. We compared its hepatic mechanism of lipid-lowering to simvastatin and explored the hormone sensitive lipase (HSL) translocation in hepatic fat loss. Oral KMUP-1 HCl (1, 2.5, 5 mg/kg/day) and simvastatin (2.5 mg/kg/day) were administered in C57BL/6J male mice fed a high-fat-diet by gavage for 8 weeks. KMUP-1 inhibited HFD-induced plasma and liver TG, total cholesterol and LDL, increased HDL/HMGR/ROCKII/PPARγ/ABCA1 expression and decreased liver-/body weight gain in the liver of mice treated with HFD for 8 weeks. After drinking KMUP-1 HCl (2.5 mg/200 ml water) for 1-14 or 8-14 weeks, decreased HFD-induced liver/body weight and increased SR-B1, PKA/PKG expression and LDL receptors (LDLRs)/PKA immunoreactivity. In HepG2 cells incubated with serum or exogenous mevalonate, KMUP-1 (10⁻⁷-10⁻⁵ M) reversed the expression of HMG CoA reductase by feed back regulation, co-localized ABCA1/ApoA-I/LXR/PPARγ and exogenous GGPP-/FPP-induced inactivation of RhoA/ROCK II. cGMP antagonist reversed KMUP-1-induced ROCK II inactivation, indicating involving cGMP/eNOS. KMUP-1 inceased PKG and LDLRs surrounded by LDL and restored oxidized LDL-decreased PKA. KMUP-1 couldn't, but simvastatin could, significantly inhibit ¹⁴C mevalonate formation. KMUP-1 could, but simvastatin couldn't, inactivate ROCK II by exogenous FPP/CGPP KMUP-1 improves HDL via increasing PPARγ/LXR/□ ABCA1/Apo-I and inhibiting SR-B1; KMUP-1 also increases LDLRs/PKA/PKG/HSL, leading to removing LDL via LDLRs and hydrolysis of TG via activated HSL. Therefore, we would consider the multiple factors reducing the obesity via improvement of PPARγ/SR-B1/LDLRs/PKA/ PKG/HSL by KMUPs-RX complex compound.

### Materials and Methods:

### Animals and serum:

In the 8 week experiment, C57BL/6J male mice (21-22 g), after fasting, were fed a HFD as a model of hyperlipidemia for 8 weeks. During the 2 month experiments, mice fasted for one night before the experiment was changed from a standard diet (STD) to a HFD and then the mice were randomly divided into 5 groups, including 2 control and 3 treatment groups. Six mice were used in each group. The control mice received either STD or HFD and the protection group was fed HFD with KMUP-1 HCl (2.5 and 5 mg/kg/day), simvastatin (5 mg/kg/day) or a target drug administered by gavage to assess weight gain followed by biochemical analysis. Liver from the protection group supplemented with KMUP-1 HCl (1 mg/kg/day) were used to determine the HMG CoA reductase expression.

In the 14-week experiment, mice were fed a HFD from week 1 to week 14. During the 3 month experiments, KMUP-1 HCl (2.5 mg) was added to 200 ml tap water and) and drink for 14 weeks or for the last 6 weeks on mice or obese mice fed HFD, separately. 5 mice had free access to drinking water from week 1 to week 14 (protective group) or from week 8 to week 14 (treatment group). Tap water was used to normalize mineral nutrition (Scheme A).

Animals were housed in the animal center with a day-night cycle system at Kaohsiung Medical University. All procedures and protocols were approved by the Animal Care and Use Committee at Kaohsiung Medical University and complied with the Guide for the Care and Use of Laboratory Animals published by the US National Institutes of Health.

### Biochemical analysis of serum:

Within 2 months, the 3 day food intake average for each animal was measured. The weight gain and plasma lipid levels of each group were determined and compared to the non-treatment control group. Mouse blood was collected during the daytime by cardiac puncture followed by centrifugation at 90 g (Benchtop Centrifuge, U.S.A.) to separate the serum, and frozen at -80°C, for biochemical analysis using a Hitachi Clinical Analyzer 7070 (Hitachi High-Technologies Co. Tokyo, Japan). Agents used in the assays were obtained from Merck & Co. (Kenilworth, NJ, U.S.A.). Triglyceride (TG), total cholesterol, HDL cholesterol and LDL cholesterol in mouse serum were measured by methods used in the clinic. To measure the hepatic TG, isolated liver were cut into small chips.

### Cell culture:

The HepG2 hepatoma cell line was purchased from the American Type Culture Collection (ATCC; Manassas, VA, U.S.A.). Cells were cultured in DMEM. The culture media was supplemented with 5% heat-inactivated FBS, penicillin (100 U/mL) and streptomycin (100 µg/mL). Cells were grown in a humidified atmosphere containing 5% CO₂ at 37°C, in which the oxygen tension in the incubator was held at 140 mmHg (20% O₂, v/v; normoxic conditions). KMUP-1 HCl dissolved in distilled water or simvastatin in a vehicle (propylene glycol) was incubated with the cells for 24 hours, followed by protein extraction. The final concentration Vehicle- or distilled water-treated cells were added with simvastatin or KMUP-1 HCl, respectively, to observe the changes of propylene glycol HMG CoA reductase expression in the medium and never exceeded 0.1 %.HepG2 cells.

### Western blotting analysis of protein expression in HepG2 cells and liver:

HepG2 cells were treated with various concentrations of drugs for 24 hours. Reactions were terminated by washing twice with cold PBS, and the cells were then harvested. Proteins in the whole-cell lysate were homogenized in an ice-cold lysis buffer and a protease inhibitor (Sigma-Aldrich, St. Louis, MO, U.S.A.). The homogenate was centrifuged at 90, 000 g for 15 minutes at 4°C, and supernatant was recovered as the total cellular protein. Cytosolic and membrane fractions of HepG2 cells were prepared using a CNM (Cytosol Nuclear Membrane) compartment protein extraction kit (BioChain Institute Inc., Hayward, CA, U.S.A.) according to the manufacturer's instructions. All of the fractionated protein solutions were stored at -80°C until analysis. To measure the expression levels of proteins by drugs, the total cell protein was extracted after incubation with treatments for 24 hours and then Western blotting analysis was performed as described previously.

For the expression of SR-B1, HMGR, PPARγ and ROCK II, isolated liver tissues were cut into small chips and placed into an extraction buffer (Tris 10 mM, pH 7.0, NaCl 140 mM, PMSF 2 mM, DTT 5 mM, NP-40 0.5%, pepstatin A 0.05 mM and leupeptin 0.2 mM) for hepatic protein extraction, and centrifuged at 20,000 g for 30 minutes. The obtained protein extract was boiled to a ratio of 4:1 with a sample buffer (Tris 100 mM, pH 6.8, glycerol 20%, SDS 4% and bromophenol blue 0.2%). Electrophoresis was performed using 10% SDS-polyacrylamide gel (1 hour, 100 V, 40 mA, 20 µg protein per lane).

Separated proteins, after 3 repeated centrifugations to discard up-layer tissue lipid inpurity, were transferred to PVDF membranes treated with 5% fat-free milk powder to block the nonspecific IgGs (90 minutes, 100 V) and incubated for 1 hour with specific protein antibody. The blot was then incubated with anti-mouse or anti-goat IgG linked to alkaline phosphatase (1:1000) for 1 hour.

### HMG CoA reductase activity and [¹⁴C]mevalonate formation:

Human recombinant HMG CoA reductase expressed in E. coli (H7039, Sigma-Adrich, Mo, U.S.A.) was used. Human recombinant HMG-CoA reductase, examined by SDS-PAGE to be ≥90% in purity, 2-8 units/mg protein in activity and ~76kDa in molecular weight (H7039, Sigma-Adrich, St. Louis, MO. U.S.A.), was used for determining the formation of [¹⁴C]mevalonate. KMUP-1 and simvastatin or vehicle were pre-incubated with 35 ng/ml enzyme in phosphate buffer pH 7.5 for 15 minutes at 37°C. The reaction was initiated by the addition of 2.5 µM [¹⁴C]HMG-CoA for another 20 minutes incubation period and terminated by further addition of 1 N HCl. An aliquot was removed by column and counted to determine the amount of [¹⁴C]mevalonate formed (ricera^{R} Co. Ltd., Taipei, Taiwan).

### cGMP pathway and RhoA/ROCK II:

To confirm that RhoA antagonist C3 exoenzyme (5 µg/ml) and ROCK antagonist Y27632 (10 µM), dissolved in 10% popyleneglycol, can inactivate_the expression of ROCK II, they were added to cells in culture for 24 hours, respectively, to measure the expression of ROCK II and related expressions of PPARγ and ABCA-1 in HepG2 cells.

To confirm that the cGMP antagonist Rp-8-pCPT-cGMPs (10 µM), dissolved in 10% propylene glycol can increase the expression of ROCK II and that KMUP-1 can reduce Rp-8-pCPT-cGMPs-induced expression of ROCK II, HepG2 cells were pre-incubated with Rp-8-pCPT-cGMPs for 30 minutes as a control and then in combination with KMUP-1 (10 µM) for 24 hours.

### Immunohistochemistry (IHC) straining of LDLRs in liver:

Liver tissues were fixed in 10% buffered formalin for 24 hours and then embedded in paraffin. The paraffin-embedded liver tissue sections (4 µm thick) were first heat immobilised and deparaffinised using xylene and then rehydrated in a graded series of ethanol, followed by a final wash in distilled water. Finally, tissue sections were stained with PAS and Mayer's hematoxylin solution.

For IHC of hepatic LDLRs in the animals that drink the KMUP-1 HCl (2.5 mg/200 mL for 1-14 weeks or 8-14 weeks), antigen retrieval of de-paraffinated sections was performed in Dako target retrieval solution, pH 9.0, in a vegetable steamer followed by the quenching of endogenous peroxidase activity with 3.0% H₂O₂ in methanol. Sections were then incubated with specific primary antibodies overnight at 4 °C in a humidified chamber. The sections were then examined using a DAKO REAL EnVision™ Detection System kit (DAKO, Carpinteria, CA, U.S.A.) and counterstained with hematoxylin. Images were obtained through a Nikon Eclipse TE200-S microscope.

### Expression and fluorescence staining of LDLRs in the presence of exogenous LDL:

HepG2 cells were used to determine the cellular protein expression of LDLRs in the presence of exogenous LDL (500 µg/mL). Bodipy-493/503 (green) and LDLRs on HepG2 cells were detected with a secondary anti-body conjugated to Cy3 (red) overnight at 4°C, followed by merger of obtained BODIPY-and LDL-images to analyze the location of LDLRs. All images were collected and analyzed by scanning with Nikon Eclipse TE200-S microscope (Tokyo, Japan).

### Expression of PKA/PKG and immunoreactivity of PKA/HSL:

To determine that KMUP-1 can affect PKA, we incubated KMUP-1 (10⁻⁴, 10⁻⁵, 10⁻⁶M) or simvastatin (10⁻⁵M) with HepG2 cells for 24 hours to measure the protein expressions of PKA/PKG by Western blotting or PKA/PKG and HSL immunoreactivities by fluorescence staining, combined with image scanning in the absence or presence of oxidized LDL (200 µg/mL).

### Measurement of hepatic oil globulet diameter:

The diameter of the white bar in the photograph was standardized at 100 µM for measuring the specific diameter of oil globulets in a whole liver slice, observed by microscope, with the aid of Powerpoint PC computer software (Microsoft^{R}, USA) and printed out by a Hewlett-Packard^{R} printer (USA). An increase in oil globulet dimeter and cell number indicated increasing liver steatosis, i.e. steaotohepatitis. In contrast, decrease in diameter and the number of fat cells indicated the inhibition of steaotohepatitis. Oil globulets on liver slices were observed with a Nikon Eclipse TE200-S microscope. Hematoxylin-eosin (H&E) and IHC staining of liver were used to assess oil globulet numbers under a microscope.

### Hematoxylin-Eosin (H&E) straining of liver and epididymal fat pads:

Mice liver and epididymal fat pads were cut and soaked in formalin, dehydrated through graded alcohols and embedded in paraffin. Specimens of liver tissues and epididymal fat pads fixed with formalin (4 %) were embedded in paraffin for one hour at 4°C, cut into 4-µm-thick sections, and subjected to H&E staining before examination by light microscope. In heart tissues, the 4-µm -thick paraffin sections were cut from paraffin-embedded tissue blocks and de-paraffinized by immersion in xylene and rehydrated. The slices were then dyed with H&E. After gently rinsing with water, each slide was dehydrated through graded alcohols and finally soaked in xylene twice.

### IHC staining of MMP-9/TNFa, HSL/p-HSL/ATGL and eNOS/IL-10:

For IHC of MMP-9/TNFα, HSL/p-HSL/ATGL and eNOS/IL-10, antigen retrieval of de-paraffinated sections was performed in Dako target retrieval solution, pH 9.0, in a vegetable steamer followed by the quenching of endogenous peroxidase activity with 3.0% H₂O₂ in methanol. Sections were then incubated with specific primary antibodies overnight at 4 °C in a humidified chamber. The antibodies used included rabbit polyclonal anti-eNOS (1:50 dilution) (Abcam, Cambridge, UK) or rabbit monoclonal anti-cleaved caspase-3 (1:50 dilution) (Cell Signaling, USA). The sections were then examined using a DAKO REAL EnVision^{™} Detection System kit (DAKO, Carpinteria, CA) and counterstained with hematoxylin. Images were obtained through a Nikon Eclipse TE200-S microscope.

### Measurement of oil globulet diameter and number in steatohepatitis:

Hematoxylin-eosin (H&E) and IHC staining of liver were used to assess oil globulet numbers under a microscope. A 100 µM white bar was used to determine oil globule sizes in the liver. Oil globulets in a cell were counted from larger to smaller until size limitations were reached. Increases of oil globulet diameter and number indicated increasing liver steatosis. In contrast, a decrease in oil globulet diameters and numbers could indicate the inhibition of liver steatosis. Oil globulets were measured using a Nikon Eclipse TE200-S microscope, aided by Powerpoint computer software (Microsoft^{R}) and printed by a HP^{R} printer to measure the diameter and number of oil globulets on paper. Counting of oil globulets was performed from larger to smaller ones until the observation reached its limit.

### Materials and reagents:

Immunoreactive bands were visualized using horseradish peroxidase-conjugated secondary antibodies and subsequent ECL detection (GE Healthcare Bio-Sciences Corp., Piscataway, NJ, U.S.A.). Mouse or rabbit monoclonal antibody for ROCK II (Upstate, Lake Placid, NY, U.S.A.), RhoA (Santa Cruz Biotechnology, Santa Cruz, CA, U.S.A.), HMG CoA reductase (Upstate, Lake Placid, NY, U.S.A.), PPARγ (Abcam, Cambridge, UK), ABCA-1 (Cell Signaling, Boston, MA, U.S.A.), ApoA-I (Abcam, Cambridge, UK), LXRα (Santa Cruz Biotechnology, Santa Cruz, CA, U.S.A.), LDLR (Abcam, Cambridge, UK), HSL (Cell Signaling, Boston, MA, U.S.A.), eNOS (Abcam, Cambridge, UK), MMP-9 (Abcam, Cambridge, UK), TNFα □ (Abcam Cambridge, UK), IL-10 (Abcam, Cambridge, UK) and the loading control protein β-action (Sigma-Adrich, St. Louis, MO, U.S.A.) were used in our Western blot analyses. Rabbit polyclonal antibody was used to recognize both PPARγ1 □ and PPARγ2 □ in the experiments. Rp-8-pCPT-cGMPs and C3 exoenzyme were purchased from Sigma-Adrich (St. Louis, MO, U.S.A.). HFD was a basal purified diet (W/60% energy from fat, Blue:58G9 Test Diet; Richmond, VA, U.S.A.). LDL was purchased from Abcam (Cambridge, UK). Oxidized LDL (oxLDL) was purchased from Biomedical Technologies Inc. (Stoughton, MA, USA). Human recombinant HMG-CoA reductase (H7039, Sigma-Adrich, St. Louis, MO. U.S.A.), examined by SDS-PAGE to be ≥90% in purity, 2-8 units/mg protein in activity and ~76kDa in molecular weight was used to determine the formation of [¹⁴C]mevalonate.

### Statistical evaluation:

The experimental results were expressed as means±SE. Statistical differences were determined by independent and paired Student's *t*-test in unpaired and paired samples, respectively. Whenever a control group was compared with more than one treated group, one way ANOVA or two way repeated measures ANOVA was used. When the ANOVA showed a statistical difference, the Dunnett's or Student-Newman-Keuls test was applied. A P value less than 0.05 was considered significant in all experiments. Analysis of the data and plotting of the figures were done using SigmaPlot software (Version 8.0, Chicago, IL, U.S.A.) and SigmaStat (Version 2.03, Chicago, IL, U.S.A.) run on an IBM compatible computer.

### Example 1: Preparation of chloroethylpiperazine

The mixture of 3% *p*-toluenesulphonic acid (100 mL), 1-bromo-2-chloroethane (30 mg) and 1-(2-chlorophenyl)piperazine (10 g) in xylene (300 mL), was heated to reflux (140-145°C, 20 hours.) and the progress of the reaction was monitored by TLC using a chloroform:methanol (8:2) solvent system. On completion, the reaction mass was cooled to 30°C and further chilled to 0-5°C when the product crystallized into off-white crystals (7.8 g).

### Example 2: Preparation of 7-[2-[4-(2-chlorophenyl) piperazinyl] ethyl]-1,3-dimethylxanthine HCl complex (KMUP-1 HCl complex)

The mixture of chloroethylpiperazine (30 mg), 3% p-toluenesulphonic acid (100 mL) and xanthine (10 mg) in acetonitrile (300 mL) was refluxed at 80-82°C for 20 hours. The Progress of the reaction was monitored by Thin layer chromatography (TLC) using a chlorofoum:methanol (9:1) solvent system. On completion the reaction mass was cooled to 50°C and filtered. The acetonitrile was recovered through atmospheric distillation (∼80 %) and toluene (300 mL) was added to the residual reaction mass and a clear solution was obtained. The toluene solution was further washed twice with 20% sodium hydroxide solution (2x50 mL) followed by 2% brine solution (2x 50 mL) at 50°C. To the toluene solution containing the product as a base, was added an isopropyl alcohol HCl solution (15%, 80 mL) and the pH was adjusted to between 2-2.5 when the salt began precipitating. The precipitated hydrochloride salt of the target molecule was isolated by filtration and re-crystallized from methanol to achieve the white crystalline KMUP-1 HCl complex (7.4 g).

### Example 3: Preparation of 7-[2-[4-(2-chlorophenyl)piperazinyl] ethyl]-1,3-dimethyl-xanthine HCl complex (KMUP-1 HCl complex)

KMUP-1 (8.0 g) was dissolved in a mixture of ethanol (10 mL) and 1N HCl (60 mL) and reacted at 50 °C for 10 minutes. The methanol was added to the solution under room temperature and the solution was incubated overnight for crystallization. The crystals were filtrated to obtain the precipitate of the KMUP-1 HCl complex (7.4 g).

### Example 4: Preparation of 7-[2-[4-(4-nitrobenzene)piperazinyl] ethyl]-1,3-dimethyl xanthine HCl complex (KMUP-3 HCl complex)

KMUP-3 (8.3 g) was dissolved in a mixture of ethanol (10 mL) and 1N HCl (60 mL). The solution was reacted at 50 °C for 20 minutes, the methanol was added thereto under room temperature, and the solution was incubated overnight for crystallization and filtrated to obtain KMUP-3 HCl complex (6.4 g).

### Example 5: Preparation of KMUP-1-γ-Polyglutamic acid complex

Method 1: Sodium γ-polyglutamic acid (20 g) was suspended in distilled water and added to KMUP-1 HCl (16 g) dissolved in 100 ml of methanol to reflux in a three-neck reactor equiped with a condenser for 1 hour. After cooling, the obtained precipitate was dissolved in 100 ml of methanol and the resulting solution was incubated for crystallization and filtrated to obtain KMUP-1-γ-Polyglutamic acid complex (35.6 g).

Method 2: KMUP-1 HCl (16 g) was dissolved in 100 ml of methanol and added to sodium alginic acid 20 g dissolved in 100 ml of methanol to reflux in a three-neck reactor equiped with a condenser for 1 hour. After cooling, obtained precipitate was filtrated and re-crystallized with 100 ml of methanol to obtain the KMUP-1-γ-Polyglutamic acid complex (35. 8 g).

### Example 6: Preparation of KMUP-3-Nicotinic acid complex

KMUP-3 (8.3 g) was dissolved in a mixture of ethanol (10 mL) and Nicotinic acid (2.4 g). The solution was reacted at 50 °C for 20 minutes, the methanol was added thereto under room temperature, and the solution was incubated overnight for crystallization and filtrated to obtain the KMUP-3-Nicotinic acid complex (8.3 g).

### Example 7: Preparation of KMUP-1-Simvastatinic complex

KMUP-1 (8.0 g) was dissolved in a mixture of ethanol (10 mL) and HCl (1 N, 60 mL) and reacted at 50 °C for 10 minutes, the methanol was added thereto under room temperature, and the solution was incubated overnight for crystallization and filtrated to obtain KMUP-1 HCl (7.4 g). KMUP-1 HCl salt (4.4 g) was then redissolved in ethanol (150 mL) for use.

In a flask equipped with a magnetic stirrer, simvastatin (4.2 g) was dissolved in a mixture of ethanol (50 mL), an aqueous solution of sodium hydroxide (4 g/60 ml) and the above-mentioned filtrate of KMUP-1 HCl salt reacted with the ethanol was added to react under room temperature. The mixture was reacted at 50 °C for 20 minutes, rapidly filtrated and incubated one hour for crystallization to obtain the KMUP-1-Simastatinic complex.

### Example 8: Preparation of KMUP-2-polyacrylic acid complex

KMUP-2 HCl (8 g) was dissolved in a mixture of methanol (100 mL) and sodium polyacrylic acid (2.5 g). The solution was refluxed in a three-neck reactor equipped with a condenser, for 1 hour. After cooling, the obtained precipitate was re-dissolved in 100 ml of methanol, and the resulting solution was incubated for crystallization and filtrated to obtain the KMUP-2-polyacrylic acid complex (7.4 g).

### Example 9: Preparation of KMUP-1-ascorbate complex from KMUP-1 base and L-ascorbic acid.

In a flask equipped with a magnetic stirrer, KMUP-1 base (13.2 g) was dissolved in a mixture of ethanol (100 mL) and an ethanol solution of equal mole ascorbic acid was added to react at 50°C for 20 minutes. After cooling, white precipitate was obtained and the sodium chloride was removed by filtration. The solvent methanol (100 mL) was added to resolve the precipitate under room temperature and incubated overnight for re-crystallization. The KMUP-1-ascorbate complex compound (16.8 g) was obtained after filtering the crystals.

### Example 10: Preparation of KMUP-2-oleate complex

8.5 g of sodium oleic acid was suspended in distilled water and added to 12.1 g of KMUP-2 HCl dissolved in 100 ml of methanol to reflux in a three-neck reactor equipped with a condenser for 1 hour. After cooling, the obtained precipitate was dissolved in 100 ml of methanol and the resulting solution was incubated for crystallization and filtrated to obtain the KMUP-2 oleate complex (16.3g).

### Example 11: Preparation of KMUP-3 ascorbate complex

KMUP-3 HCl (8.5 g) was dissolved in 100 ml of ethanol and added to 3.9g of sodium ascorbic acid and refluxed in a three-neck reactor equipped with a condenser for 1 hour. After cooling, the obtained precipitate was filtrated and re-crystallized with 100 ml of methanol to obtain the KMUP-3 ascorbate complex (9.7 g).

### Example 12: Preparation of Sildenafil-CMC complex

3.6 g of sodium sodium carboxyl methylcellulose was suspended in distilled water for use.

In a flask equipped with a magnetic stirrer, 13.2 g of Sildenafil citrate was dissolved in a mixture of ethanol (100 mL) and water (30 mL), and an ethanol solution of sodium CMC was added and reacted at 50°C for 20 minutes. After cooling, a white precipitate was obtained and the sodium citrate was removed by filtration. The solvent 100 mL of methanol was added to resolve the precipitate under room temperature and incubated overnight for re-crystallization. The Sildenafil-CMC complex (10.4 g) was obtained after filtering the crystals.

### Example 13: Preparation of the composition in tablets

Tablets were prepared using standard mixing and formulation techniques as described in U.S. Pat. No. 5, 358, 941, to Bechard et al., issued Oct. 25, 1994, which is incorporated by reference herein in its entirety.

| | |
|---|---|
| KMUP-3-citric acid complex | 100 mg |
| Lactose | qs |
| Com starch | qs |

### Example 14: Preparation of the composition in tablets

Tablets were prepared using standard mixing and formulation techniques as described in U.S. Pat. No. 5, 358, 941, to Bechard et al., issued Oct. 25, 1994, which is incorporated by reference herein in its entirety.

| | |
|---|---|
| Sildenafil-oleate complex | 50 mg |
| Lactose | qs |
| Com starch | qs |

### Embodiments

1. A method for inhibiting a liver disease, characterized by comprising steps of: providing a subject suffering from the liver disease; and administering one of a KMUPs complex compound represented by one of formula II and formula III, and a pharmaceutical composition thereof to the subject in a dosage between 1 and 5.0 milligrams per kilogram of body weight, 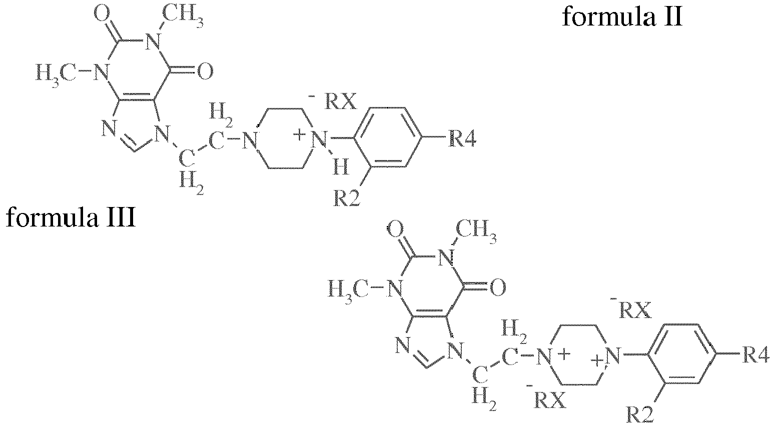 characterized in that: R2 and R4 are each selected independently from the group consisting of a C1-C5 alkoxy group, hydrogen atom, nitro group, and a halogen atom; RX includes a carboxylic group selected from the group consisting of Statin analogue, co-polymer, poly-γ-polyglutamic acid derivative, ascorbic acid, oleic acid, phosphoric acid, citric acid, nicotinic acid and sodium carboxymethyl cellulose (sodium CMC).
2. A method of providing a medical effect for inhibiting liver disease, characterized by comprising steps of providing a subject in need thereof; and administering an effective amount of pharmaceutical composition of a KMUPs derivative to the warm-blooded animal in need thereof.
3. The method of Embodiment 2, characterized in that the administration is performed by one selected from an oral, injection, inhalation and topical administration.
4. The method of any one of Embodiments 2-3, characterized by comprising a step of: combination administrating a pharmaceutically effective amount of a compound of KMUPs derivative and a statins analogues to the subject in need thereof.
5. The method of any one of Embodiments 2-4, characterized in that the statins analogues is one selected from the group consisting of Atorvastatin, Cerivastatin, Fluvastatin, Lovastatin, Mevastatin, Pravastatin, Rosuvastatin, Simvastatin and Pravastatin acid.
6. The method of any one of Embodiments 2-5, characterized in that the Co-polymers are one selected from the group consisting of hyaluronic acid, polyacrylic acid, polymethacrylates (PMMA), Eudragit, dextran sulfate, heparan sulfate, polylactic acid or polylactide (PLA), polylactic acid sodium (PLA sodium) and polyglycolic acid sodium (PGCA sodium).
7. The method of any one of Embodiments 2-6, characterized in that the Poly-γ-polyglutamic acid (γ-PGA) derivative is one selected from the group consisting of an alginate sodium, poly-γ-polyglutamic acid sodium (γ-PGA sodium), poly-γ-polyglutamic acid (γ-PGA) and an alginate-poly-lysine- alginate (APA).
8. A non-alcoholic fatty liver disease prevention pharmaceutical composition including: 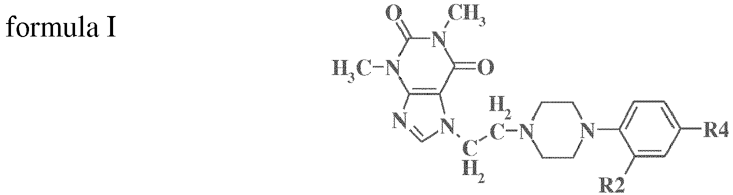 an effective amount of a compound of formula I, characterized in that R₂ and R₄ are each selected independently from the group consisting of a C1-C5 alkoxy group, hydrogen atom, nitro group, and a halogen atom; and a pharmaceutically acceptable carrier.
9. The non-alcoholic fatty liver disease prevention pharmaceutical composition of Embodiment 8, characterized in that the compound is a treatment for non-alcoholic fatty liver disease and reduces high-fat diet-induced accumulation of fat in the liver.
10. A combination method for inhibiting a liver disease, characterized by comprising steps of: providing a subject suffering from the liver disease; and administering a combination of one selected from the group consisting of Piperazinyl Analogs compounds, and administrating a pharmaceutically effective amount of a compound selected from the group consisting of a Statin analogue, co-polymer, poly-γ-polyglutamic acid derivative, ascorbic acid, oleic acid, phosphoric acid, citric acid, nicotinic acid and sodium carboxymethyl cellulose (sodium CMC), thereof to the subject in need thereof in a combination dosage between 1 and 5.0 milligrams per kilogram of body weight.
11. The combination method of Embodiment 10, characterized in that Sildenafil Analogs compounds include Sildenafil, Hydroxyhomosildenafil, Desmethylsildenafil, Acetidenafil, Udenafil, Vardenafil and Homosildenafil.
12. A combination method for inhibiting liver disease, characterized by comprising a step of: combination administrating a pharmaceutically effective amount of a compound of Sildenafil Analogs derivative and a different active agent to the subject in need thereof.
13. The combination method of Embodiment 12, characterized in that the compound providing a subject suffering from the liver disease, involves the beneficial effects of removing plasma LDL via increasing LDLRs, increasing circulation and hepatic fat loss via HSL which is around the lipid droplets of adipocytes in the entire body and the sites of lipid storage in hepatic cells.
14. The combination method of any one of Embodiments 12-13, characterized in that the liver disease comprises one selected from a group consisting of an non-alcoholic fatty liver disease, hyperadiposity and reduces high-fat diet-induced accumulation of fat in the liver and a combination thereof.
15. The combination method of any one of Embodiments 12-14, characterized in that the warm-blooded animal includes man, goat, lamb, pig, cow, chicken, duck.
16. A combination method for inhibiting liver disease, characterized by comprising a step of: combination administrating a pharmaceutically effective amount of a compound of Piperazinyl Analogs compound and different active agent to the subject in need thereof.
17. The combination method of Embodiment 16, characterized in that the Piperazinyl Analogs compound selected from a group consisting of KMUPs, Sildenafil Analogs.
18. The combination method of Embodiments 16-17, characterized in that Sildenafil Analogs compounds include Sildenafil, Hydroxyhomosildenafil, Desmethylsildenafil, Acetidenafil, Udenafil, Vardenafil and Homosildenafil.
19. The combination method of Embodiments 16-18, characterized in that the compound providing a subject suffering from the liver disease, involved in the beneficial effects of hepatic PPAR □ agonist, decreasing LDL-associated lipid metabolism.
20. The combination method of Embodiments 16-19, characterized in that the compound providing a subject suffering from the liver disease, involves the beneficial effects of removing plasma LDL via increasing LDLRs, increasing circulation and hepatic fat loss via HSL which is around the lipid droplets of adipocytes in the entire body and the sites of lipid storage in hepatic cells.
21. The combination method of Embodiments 16-20, characterized in that the liver disease comprises one selected from a group consisting of an non-alcoholic fatty liver disease, hyperadiposity and reduces high-fat diet-induced accumulation of fat in the liver and a combination thereof.
22. The combination method of Embodiments 16-21, characterized in that the administration is performed by one selected from an oral, an injection, an inhalation and a topical administration.
23. A method, characterized by comprising steps of providing a subject in need thereof; and administering one selected from the group consisting of a Sildenafil Analogs derivative compound and Sildenafil Analogs-RX complex compound, pharmaceutically acceptable salts thereof; and a pharmaceutical composition thereof to the subject in a oral dosage of 1 to 5.0 milligrams per kilogram of body weight of animals.
24. A method for inhibiting a liver disease, characterized by comprising steps of: providing a subject suffering from the liver disease; and administering a pharmaceutical composition of a KMUPs complex compound selected from the group consisting of KMUPs-RX complex compound and KMUPs-RX- RX complex compound and administer to the subject a dosage between 1 and 5.0 milligrams per kilogram of body weight, characterized in that KMUPs include KMUP-1, KMUP-2, KMUP-3 and KMUP-4; RX includes a carboxylic group selected from the group consisting of a Statin analogue, a poly-γ-polyglutamic acid derivative, a co-polymer, a ascorbic acid, an oleic acid, a phosphoric acid, a citric acid, a nicotinic acid and a sodium CMC.

### References:

1. Dai ZK, et al.. (2014) Xanthine Derivative KMUP-1 Reduces Inflammation and Hyperalgesia in a Bilateral Chronic Constriction Injury Model by Suppressing MAPK and NFκBActivation. Mol Pharm., 11, 1621-1631.
2. Bivalacqua TJ, et al. (2013) Sildenafil citrate-restored eNOS and PDE5 regulation in sickle cell mouse penis prevents Priapism via control of oxidative/nitrosative stress. PLoSONE 8: e68028. doi:10.1371/journal. pone.0068028.
3. Chung HH, et al. (2010) The xanthine derivative KMUP-1 inhibits models of pulmonary artery hypertension via increased NO and cGMP-dependent inhibition of RhoA/Rho kinase. Br J Pharmacol 160: 971-986.
4. Chung HH, et al. (2010) KMUP-1 inhibits pulmonary artery proliferation by targeting serotonin receptors/transporter and NO synthase, inactivating RhoA and suppressing AKT/ERK phosphorylation. Vascular Pharmacology 53: 239-249.

## Claims

1. A method for inhibiting a liver disease, **characterized by** comprising:
administering a pharmaceutical composition of one of a piperazine analogue and a piperazine analogue complex to a warm-blooded animal suffering from the liver disease.

2. The method as claimed in Claim 1, **characterized in that** the piperazine analogue is one of a KMUPs compound and a sildenafil analogue compound.

3. The method as claimed in Claim 2, **characterized in that** the KMUPs compound is one selected from the group consisting of KMUP-1, KMUP-2, KMUP-3 and KMUP-4.

4. The method as claimed in Claim 2 or 3, **characterized in that** the sildenafil analogue compound is one selected from the group consisting of Sildenafil, Hydroxyhomosildenafil, Desmethylsildenafil, Acetidenafil, Udenafil, Vardenafil and Homosildenafil.

5. The method as claimed in any one of Claims 1-4, **characterized in that** the warm-blooded animal is one selected from the group consisting of a human, a goat, a sheep, a pig, a cow, a chicken and a duck.

6. The method as claimed in any one of Claims 1-5, **characterized in that** the liver disease is one selected from the group consisting of nonalcoholic fatty liver disease, hyperadiposity, high-fat-diet-induced lipid accumulation in the liver and any combination thereof.

7. The method as claimed in any one of Claims 1-6, **characterized in that** the pharmaceutical composition acts as a peroxisome proliferator activated receptor (PPAR) agonist, lowers plasma low-density of lipoprotein (LDL) by increasing low density lipoprotein receptor (LDLRs) function and facilitates fat loss by facilitating hormone-sensitive lipase (HSL) function.

8. The method as claimed in any one of Claims 1-7, **characterized in that**:
the piperazine analogue complex consists of a KMUPs complex (KMUPs-RX) compound and a sildenafil analogue complex (sildenafil analogue-RX) compound; and
RX is a different active agent.

9. The method as claimed in Claim 8, **characterized in that** the different active agent is one selected from the group consisting of a statin analogue, a co-polymer, a γ-polyglutamic acid (PGA) derivative, an ascorbic acid, an oleic acid, a phosphoric acid, a citric acid, a nicotinic acid and a sodium carboxymethylcellulose (sodium CMC).

10. The method as claimed in Claims 1-9, **characterized in that** the statin analogue is one selected from the group consisting of an atorvastatin, a cerivastatin, a fluvastatin, a lovastatin, a mevastatin, a pravastatin, a rosuvastatin, a simvastatin, a pravastatin acid and their pharmaceutically acceptable salts.

11. The method as claimed in Claim 9 or 10, **characterized in that** the co-polymer is one selected from the group consisting of a hyaluronic acid, a polyacrylic acid (PAA), a poly(methyl methacrylate) (PMMA), an EUDRAGIT^{®} polymer, a dextran sulfate, a heparan sulfate, a polylactic acid (PLA) or its sodium salt, and a sodium salt of polyglycolic acid (PGCA).

12. The method as claimed in any one of Claims 9-11, **characterized in that** the γ-polyglutamic acid (γ-PGA) derivative is one selected from the group consisting of a sodium alginate, a γ-polyglutamic acid (γ-PGA), a sodium γ-polyglutamate (sodium γ-PGA), and an alginate-poly-1-lysine-alginate (APA).

13. A pharmaceutical composition for use in inhibiting a liver disease, **characterized in that** the pharmaceutical composition comprises one of a piperazine analogue and a piperazine analogue complex.

14. The pharmaceutical composition as claimed in Claim 13, **characterized in that** the pharmaceutical composition is applied to a warm-blooded animal suffering from the liver disease.

15. The pharmaceutical composition as claimed in Claim 13 or 14, **characterized in that** the warm-blooded animal is one selected from the group consisting of a human, a goat, a sheep, a pig, a cow, a chicken and a duck.
